# EUROPEAN PATENT APPLICATION

(11) **EP 3 640 331 A1**
(43) Date of publication of application: **22.04.2020**
(21) Application number: 19165284.1
(22) Date of filing: 26.03.2019
(51) Int. Cl.: C12N 15/10, C40B 40/02

(54) **METHOD FOR ANALYZING A LIBRARY**

(30) Priority: 18.10.2018 US 201862747661 P
(71) Applicant: BASF SE, 67056 Ludwigshafen am Rhein (DE)
(72) Inventor: BLIZNYUK, Marya, San Diego, CA 92121 (US); LISZKA, Michael, San Diego, CA 92121 (US)
(74) Representative: BASF IP Association

(57) **Abstract**

The present invention is directed to methods of analyzing cell or gene libraries which are suitable for high throughput analysis of said libraries. In particular, the invention is directed to automated methods of high throughput screening using automated robotics, information technology, computers, and acoustic dispensing devices.

## Description

### Field of the Invention

The present invention is directed to methods of analyzing cell or gene libraries which are suitable for high throughput analysis of said libraries. In particular, the invention is directed to automated methods of high throughput screening using automated robotics, information technology, computers, and acoustic dispensing devices.

### Background of the Invention

High-throughput screening (HTS) refers to screening of large numbers of samples in a short time to arrive at desired molecules, and is extensively used in e.g. drug discovery, enzyme biology, and other biological, biochemical, and chemical fields. Using robotics, data processing/control software, liquid handling devices, and sensitive detectors, high-throughput screening allows for the speedy processing of millions of chemical, genetic, or pharmacological tests. HTS procedures can rapidly identify active compounds, antibodies, or genes that modulate a particular biomolecular pathway. The results of these experiments provide starting points for drug design and for understanding the interaction or role of a particular biochemical process in biology.

One example of high throughput screening (HTS) is enzyme evolution, also known as directed evolution, a method used in protein engineering that mimics the process of natural selection to steer proteins or nucleic acids toward a user-defined goal. Such a goal may for example be a certain function, improved function, or other desired quality exhibited by the enzyme. Directed evolution involves subjecting a gene to iterative rounds of mutagenesis (creating a library of gene variants), selection (recombinantly expressing those gene variants in cells, thereby creating a library of cells expressing gene variants, and isolating library members expressing gene variants with the desired function), and amplification (generating a nucleic acid template for the next round of analysis). Directed evolution is used both for protein engineering as an alternative to rationally designing modified proteins, as well as for studies of fundamental evolutionary principles in a controlled laboratory environment.

Aside from directed evolution of proteins or nucleic acids, high throughput screening can similarly be used to identify or analyze libraries of nucleic acids, such as DNAs and RNAs, peptides, polypeptides, and proteins, such as enzymes, for desired properties, all of which can be recombinantly expressed in cells to create cell libraries. Frequently used cells in high throughput screening are prokaryotes such as bacteria and archaea, and eukaryotes such as fungi (e.g. yeast), as well as animal and plant cell lines.

While high throughput screening techniques and the devices required to perform these techniques for screening biological material, cells, nucleic acids, and proteins have improved in recent years, these techniques are still complex and sometimes require human intervention to perform certain activities. For example, steps that often have to be performed manually include plating of cells, picking single colonies of cells (hit picking), growing and propagating these picked cells, as well as isolating and sequencing of DNA or proteins of interest. Such processing by humans in a manual fashion during high throughput screening procedures is expensive, slow, and prone to errors. Therefore, there is a need in the art to reduce costs, required time, and error frequency when performing high throughput screening of biological material by automation.

### Summary of the Invention

The inventors have developed a method for analyzing a library comprising, in sequence, the steps of:
(a) providing a library comprising a plurality of members, wherein each member comprises one or more cells, and wherein each member recombinantly expresses a protein of interest;
(b) arraying solutions of the members of the library in one or more microtiter plates;
(c) transferring a specified volume of each solution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the cells present in the picked colonies to multiply;
(h) isolating DNA from the cells in the liquid medium; and,
(i) sequencing the DNA encoding the protein of interest.

The inventive method provides improved high throughput screening for proteins that reduces cost, time and error frequency compared to manual or semi-automated methods of the art. Specifically, the automated colony picking and dilutions reduce the time required to prepare samples for sequencing. The automated acoustic plating onto an agar tray eliminates the need for human pipetting, thereby reducing errors and processing time. In addition, the automated acoustic plating also enables control over the grid size and spacing of the spots to a degree that manual pipetting cannot reliably achieve. Finally, the automated colony picking can recognize areas in which multiple dilutions have been plated as one region, i.e. as all belonging to the same library member, and subsequently identify and pick a predefined number of single colonies from this region. This method of colony picking improves accuracy by picking single colonies, i.e. colonies that arose from single cells rather than multiple cells potentially recombinantly expressing different proteins. This enables more accurate sequencing of the expressed protein.

In an embodiment, the protein is an enzyme, a peptide, an antibody or antigen-binding fragment thereof, a protein antibiotic, a fusion protein, a vaccine or a vaccine-like protein or particle, a growth factor, a hormone, or a cytokine. In one such embodiment, the protein is an enzyme, preferably the enzyme is selected from the group consisting of phytase, protease, beta-glucanase, xylanase, mannanase, lipase, cellulase, glucoamylase, amylase, alpha-amylase, and beta-amylase.

In an embodiment, the one or more cells are selected from the group consisting of prokaryotic cells and eukaryotic cells. In one such embodiment, the one or more cells are prokaryotic cells and the prokaryotic cells are bacterial cells. In another such embodiment, the one or more cells are eukaryotic cells, and the eukaryotic cells are selected from the group consisting of fungal cells, animal cells, and plant cells.

In an embodiment, the one or more cells are selected from the group consisting of *E*. *coli* cells, *Bacillus* cells, *Trichoderma* cells, *Komagataella* cells, *Aspergillus* cells, *Thermothelomyces* cells, and *Saccharomyces* cells.

In an embodiment, the protein has a desired function selected from the group consisting of an enzymatic activity, a substrate specificity, an enzymatic stability, an expression titer, and any combination thereof.

In an embodiment, the one or more microtiter plates comprise wells, optionally wherein the number of wells is selected from the group consisting of 6, 12, 24, 48, 96, 384, and 1536.

In an embodiment, in step (b) of the inventive method, one or more dilutions of the solutions are prepared before the members are arrayed, optionally wherein the dilutions are serial dilutions, wherein the solutions and the dilutions are arrayed in step (b).

In an embodiment, the solutions and/or dilutions of the solutions comprise a liquid comprising a glycerol stock.

In an embodiment, the specified volume transferred from the one or more microtiter plates onto the one or more agar trays using the acoustic dispenser in step (c) of the inventive method is 1 nL, 2 nL, 3 nL, 4 nL, 5 nL, 6 nL, 7 nL, 8 nL,9 nL, 10 nL, 11 nL, 12 nL, 13 nL, 14 nL, 15 nL,16 nL, 17 nL, 18 nL, 19 nL, 20 nL, 21 nL, 22 nL, 23 nL, 24 nL, 25 nL, 26 nL, 27 nL, 28 nL, 29 nL or 30 nL.

In an embodiment, in step (c) of the inventive method, the one or more agar trays are located above the one or more microtiter plates, and the one or more agar trays are oriented such that their agar surface faces the one or more microtiter plates.

In an embodiment, the incubation time in step (d) of the inventive method is from 12 hours to 120 hours.

In an embodiment, the incubation temperature in step (d) of the inventive method is from 15 degrees C to 50 degrees C.

### Brief Description of the Drawings

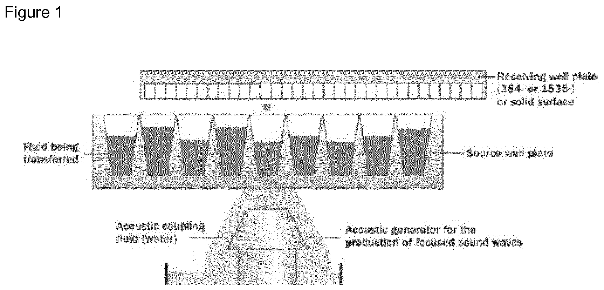

Figure 1 is a schematic describing the basic operation of an exemplary acoustic dispenser, the Acoustic Transfer System (ATS) by EDC Biosystems. A source well in a microtiter plate is centered over an acoustic horn, which generates sound energy. This energy travels through a coupling fluid, the bottom of the microtiter plate, and the solution in the well of the microtiter plate, creating a wave in the well that results in the formation of a single droplet of the solution present in the well. The receiving well or surface such as an agar tray is inverted above the source well, and the single droplet travels vertically from the source well until it lands in the corresponding receiving well or surface.
Figure 2 is an agar tray spotted with 32 members of an *E*. *coli* library. Each member is spotted in three increasing dilutions (left to right) to obtain single colonies, according to the protocol of Example 2.
Figure 3 is a close-up of one member of a *P. pastoris* library transferred to an agar tray in three increasing dilutions (left to right) according to the protocol of Example 2.
Figure 4 is an agar tray spotted with *P. pastoris* library members according to Example 3. Increasing dilutions (top to bottom) are indicated. "1x" corresponds to an OD₆₀₀ of 1.
Figure 5 shows the sequencing data for amylase genes of interest from *P. pastoris* library members according to Examples 1 and 4. The mixed culture resulted in multiple chromatogram peaks for residues 128, 256, and 433.
Figure 6 shows sequencing data from single colonies picked from the 1,000 fold dilution array of the mixed population in Figure 4 described in Examples 3 and 4, demonstrating ach of the three variants was recovered from the mixed-culture after ATS transfer and growth and that each of the three recovered variants was genetically pure.

### Detailed Description of the Invention

Although the present invention will be described with respect to particular embodiments, this description is not to be construed in a limiting sense.

Before describing in detail exemplary embodiments of the present invention, definitions important for understanding the present invention are given. Unless stated otherwise or apparent from the nature of the definition, the definitions apply to all methods and uses described herein.

As used in this specification and in the appended claims, the singular forms of "a" and "an" also include the respective plurals unless the context clearly dictates otherwise. In the context of the present invention, the terms "about" and "approximately" denote an interval of accuracy that a person skilled in the art will understand to still ensure the technical effect of the feature in question. The term typically indicates a deviation from the indicated numerical value of ±20 %, preferably ±15 %, more preferably ±10 %, and even more preferably ±5 %.

It is to be understood that the term "comprising" is not limiting. For the purposes of the present invention the term "consisting of" is considered to be a preferred embodiment of the term "comprising". If hereinafter a group is defined to comprise at least a certain number of embodiments, this is meant to also encompass a group which preferably consists of these embodiments only.

Furthermore, the terms "first", "second", "third" or "(a)", "(b)", "(c)", "(d)" etc. and the like in the description and in the claims are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein, unless expressly indicated otherwise. In case the terms "first", "second", "third" or "(a)", "(b)", "(c)", "(d)", "i", "ii" etc. relate to steps of a method or use or assay there is no time or time interval coherence between the steps, i.e. the steps may be carried out simultaneously or there may be time intervals of seconds, minutes, hours, days, weeks, months or even years between such steps, unless otherwise indicated in the application as set forth herein above or below.

It is to be understood that this invention is not limited to the particular methodology, protocols, reagents etc. described herein as these may vary. It is also to be understood that the terminology used herein is for the purpose of describing particular embodiments only and is not intended to limit the scope of the present invention that will be limited only by the appended claims. Unless defined otherwise, all technical and scientific terms used herein have the same meanings as commonly understood by one of ordinary skill in the art.

The term "library" as used herein denotes a collection of members wherein at least two members of the library differ from each other in at least one feature, preferably in the nucleic acid sequence encoding the recombinantly expressed protein. In one embodiment, each member of the library differs from all other members of the library in at least one feature. Different members of the library may have the same nucleic acid sequence encoding the recombinantly expressed protein, as long as at least two members of the library differ from each other in the nucleic acid sequence encoding the recombinantly expressed protein. In a particular embodiment, each member of the library differs from all other members of the library in the nucleic acid sequence encoding the recombinant protein. In one embodiment, each member of the library recombinantly expresses a variant of a parent protein. The variant may differ from the parent protein in one or more amino acid substitutions, insertions or deletions. In one embodiment, each member of the library recombinantly expresses a different variant of a parent protein. In one embodiment, at least two members of the library differ from each other in that they recombinantly express different variants of the same parent protein. For example, member 1 may express a variant of a parent protein A comprising the amino acid substitution S134A and member 2 may express a variant of said parent protein A comprising the amino acid substitution G225C.

A library typically comprises from 1,000 to 100,000 members, from 5,000 to 80,000 members, from 10,000 to 60,000 members or from 20,000 to 50,000 members.

The library may be provided by mutagenizing the amino acid sequence of a protein which is also called parent protein, yielding different variants of the parent protein. Preferably, the amino acid sequence is randomly mutagenized. Techniques for random mutagenesis of proteins are well-known to the skilled person and include gene site saturation mutagenesis (GSSM) which is described in Kretz et al. (2004) Methods Enzymol. 388: 3-11. After mutagenesis, the variants of the parent protein may be screened for a desired function and only those variants having the desired function may be further analyzed by the method of the present invention. As discussed inmore detail further below, a desired function includes enzymatic activity, substrate specificity, enzymatic stability, and expression titer.

Within the present invention, a "member" of a library comprises one or more cells recombinantly expressing a protein. Preferably, the one or more cells of a member are obtained from the same cell clone. Preferably, the members vary only in one aspect, which aspect is preferably related to the recombinantly expressed protein. That is, the members of a library are preferably all of the same cell type, and only vary in, e.g. the sequence of the recombinantly expressed protein or the nucleic acid sequence encoding the recombinantly expressed protein.

The one or more cells that are comprised by a member or a library can be prokaryotic cells or eukaryotic cells.

Exemplary prokaryotic cells bacterial cells. Exemplary bacterial cells are *Escherichia coli* cells and *Bacillus* cells, e.g. *Bacillus subtilis* cells or *Bacillus licheniformis* cells.

Exemplary eukaryotic cells are selected from the group consisting of fungal cells, animal cells, and plant cells. Exemplary fungal cells are yeast cells, *Trichoderma* cells, e.g. *Trichoderma reesei, Aspergillus* cells, e.g. *Aspergillus niger* cells*,* and *Thermothelomyces* cells, e.g. *Thermothelomyces thermophila.* Exemplary yeast cells are *Komagataella* cells, e.g. *Komagataella phaffi* (also known as *Pichia pastoris*) cells, and *Saccharomyces* cells, e.g. *Saccharomyces cerevisiae* cells.

The term "protein" as used herein refers to a peptide, a polypeptide, an enzyme, an antibody or antigen-binding fragment thereof, a protein antibiotic, a fusion protein, a vaccine or a vaccine-like protein or particle, a growth factor, a hormone, or a cytokine. All proteins have an amino acid sequence that is encoded by a nucleic acid sequence. Proteins are produced from genes by the processes of translation and transcription, and can further be post-translationally modified. Proteins may contain tags, leader peptides, and/or other additional sequences. An "enzyme" is a protein that catalyzes a chemical reaction. Preferably, the enzyme is a hydrolase. Hydrolases are a class of enzymes that is commonly used as biochemical catalysts that utilize water to break a chemical bond. Exemplary enzymes are phytase, protease, beta-glucanase, xylanase, mannanase, lipase, cellulase, glucoamylase, amylase, alpha-amylase, and beta-amylase.

"Recombinantly expressed" proteins refer to proteins produced by recombinant DNA techniques, e.g., produced from cells transformed with an exogenous DNA construct encoding the desired protein. The recombinantly expressed proteins of different members of the same library can be functionally or structurally related. The recombinantly expressed proteins of different members of the same library may be encoded by different alleles of the same gene. The recombinantly expressed proteins of different members of the same library may be homologs or orthologs of each other. The recombinantly expressed proteins of different members of the same library may be variants of a parent protein, e.g. variants generated by directed evolution. The variants may differ from the parent protein in one or more amino acids and may comprise one or more amino acid substitutions, deletions or insertion in comparison with the parent protein.

The term "arraying" as used herein denotes arranging the members of a library in a defined two-dimensional pattern and/or order in a microtiter plate or on the surface of an agar tray. Each defined well or spot of the array is correlated with a specific member of the library. Preferably, each defined well or spot is also correlated with a dilution value of the specific member it is correlated with, with undiluted members having a dilution value of zero. That is, each well or spot can be identified as a specific member at a specific dilution. Arraying can be performed manually or by automation, e.g. by a robot. Automated arraying is preferred in the context of the present invention. Exemplary robots for automated arraying include, e.g., liquid handlers, such as the 8-channel Vantage liquid handler, by Hamilton.

The terms "microplate", "microtiter plate", "microwell plate", or "multiwell plate" are used interchangeably herein and refer to a flat plate with multiple "wells" used as small test vessels. The microtiter plate has become a standard tool in analytical research and clinical diagnostic testing laboratories. A microplate typically has 6, 12, 24, 48, 96, 384,1536, 3456, or 9600 sample wells arranged in a 2:3 rectangular matrix. Each well of a microplate typically holds a volume of from tens of nanoliters to several milliliters of liquid. Wells can be either circular or square in cross section, and have flat, round, or pointed bottoms. Microtiter plates are made of glass or plastic and can be transparent or opaque. In one embodiment, the microtiter plate is a transparent glass plate. The microtiter plate is compatible with the acoustic dispenser. Exemplary microtiter plates include, e.g., 384 Greiner 781090.

"Agar trays" are glass or plastic trays having a size that corresponds to the size of microtiter plates and are filled with a suitable culture medium solidified with agarose. The culture medium is solidified by a suitable amount of agarose so that no culture medium drops from the agar tray when the agar tray is inverted. The height of the agar within the agar tray is between 1 and 15 mm, preferably between 2 and 13 mm, more preferably between 3 and 10 mm and even more preferably between 4 and 8 mm. In one embodiment, the height of the agar within the agar tray is 1 mm, 2 mm, 3 mm, 4 mm, 5 mm, 6mm, 7 mm, 8 mm, 9 mm, 10 mm, 11 mm, 12 mm, 13 mm, 14 mm, or 15 mm. The height of the agar within the agar tray is selected such that the agar forms a substantially even surface and/or a surface which does not substantially dry during the transfer of the cells to the agar tray. Agar height can affect the quality of the single colonies formed in step (d) of the inventive method. specified volumeExemplary trays for filling with solidified medium include, e.g., Nunc OmniTrays.

"Culture medium" refers to a medium comprising any nutrients or other components necessary to support cell growth on or in the culture medium. Culture media can be liquid (referred to herein as "liquid medium"), semi-solid, or solid (e.g. liquid medium solidified with agarose, such as 0.5%, 0.8%, 1.0%, 1.3%, 1.5%, 1.8% or 2.0% agarose). The choice of culture medium will be dependent on the cell type or cell types present in the library, and is known to the skilled person from the art. The culture medium may contain a selection agent to select for those cells which contain the recombinant nucleic acid molecule from which the recombinantly expressed protein is expressed. Exemplary media for *Pichia pastoris* include, e.g., YPDwhich contains 1 % Yeast Extract, 2% Peptone and 2% Glucose.

An "acoustic dispenser" refers to a machine in which localized acoustic energy is generated at specific locations corresponding to one or more well positions in a microtiter plate below a microtiter plate. The acoustic energy displaces a specified volume of liquid from the well it was generated below. The volume typically ranges from about 1 to 30 nanoliters, e.g. 1 nL, 2 nL, 3 nL, 4 nL, 5 nL, 6 nL, 7 nL, 8 nL,9 nL, 10 nL, 11 nL, 12 nL, 13 nL, 14 nL, 15 nL,16 nL, 17 nL, 18 nL, 19 nL, 20 nL, 21 nL, 22 nL, 23 nL, 24 nL, 25 nL, 26 nL, 27 nL, 28 nL, 29 nL, or 30 nL. The displaced liquid is propelled upward by the acoustic energy, where it meets an agar tray which is positioned above the microtiter plate with the agar side facing the open wells of the microtiter plate. The distance between the microtiter plate and the agar tray may be from 1 to 20 mm, e.g. 1 mm, 2 mm, 3 mm, 4 mm, 5 mm, 6 mm, 7 mm, 8 mm, 9 mm, 10 mm, 11 mm, 12 mm, 13 mm, 14 mm, 15 mm, 16 mm, 17 mm, 18 mm, 19 mm, or 20mm. The agar tray and microtiter plate edges are aligned, so that each spot on the agar tray can be directly correlated to a well of the microtiter plate, and, as a result, with a specific library member, preferably at a specific dilution. An exemplary setup illustrating the basic operation of an acoustic dispenser is shown in Figure 1. Exemplary acoustic dispensers include, e.g., the ATS Gen5 by EDC Biosystems or the Echo Liquid Handlers from Labcyte.

The term "colonies" refers to visible clusters of cells growing on the surface of or within a solid medium. In one embodiment, the colony is derived from a single starting cell by cell division. A "single colony" refers to a colony which does not touch or overlap with neighboring colonies. The likelihood that a colony originated from a single cell rather than a plurality of cells is much higher for single colonies. Single colonies are therefore preferred for subsequent analysis, e.g. by nucleic acid sequencing, as only one form of the molecule to be analyzed is likely to be present within the single colony. To obtain single colonies, dilutions can be performed until few enough cells are present in a volume of liquid that, upon transfer onto the agar tray, colonies arising from single cells are far enough spaced apart not to touch or overlap.

"Incubating" refers to leaving an inoculated solid or liquid medium, e.g. in an agar tray or a microtiter plate, at conditions that promote cell growth and division for a period of time. The specific set of conditions, e.g. specific temperatures, humidities, CO₂ concentrations, etc., will depend on the cell type or cell types comprised in the library. In one embodiment, the incubation temperature is from 15°C to 50°C, preferably between 20°C and 45°C, more preferably between 25°C and 40°C and most preferably between 30°C and 37°C. Similarly, the period of incubation will depend on the growth and/or division rate of the cells at a given set of conditions. In one embodiment, the period of incubation is from 12 to 120 hours, more preferably from 15 to 100 hours, even more preferably from 20 to 70 hours and most preferably from 24 to 48 hours. In one embodiment, the incubation temperature is from 15°C to 50°C and the period of incubation is from 12 to 120 hours. In another embodiment, the incubation temperature is from 20°C to 45° C and the period of incubation is from 12 to 120 hours. In another embodiment, the incubation temperature is from 25°C to 40°C and the period of incubation is from 12 to 120 hours. In yet another embodiment, the incubation temperature is from 30°C to 37°C and the period of incubation is from 12 to 120 hours. In one embodiment, the incubation temperature is from 15°C to 50°C and the period of incubation is from 15 to 100 hours. In another embodiment, the incubation temperature is from 20°C to 45°C and the period of incubation is from 15 to 100 hours. In another embodiment, the incubation temperature is from 25°C to 40°C and the period of incubation is from 15 to 100 hours. In yet another embodiment, the incubation temperature is from 30°C to 37°C and the period of incubation is from 15 to 100 hours. In one embodiment, the incubation temperature is from 15°C to 50°C and the period of incubation is from 20 to 70 hours. In another embodiment, the incubation temperature is from 20°C to 45°C and the period of incubation is from 20 to 70 hours. In another embodiment, the incubation temperature is from 25 °C to 40°C and the period of incubation is from 20 to 70 hours. In yet another embodiment, the incubation temperature is from 30°C to 37°C and the period of incubation is from 20 to 70 hours. In one embodiment, the incubation temperature is from 15°C to 50°C and the period of incubation is from 24 to 48 hours. In another embodiment, the incubation temperature is from 20 °C to 45°C and the period of incubation is from 24 to 48 hours. In another embodiment, the incubation temperature is from 25°C to 40°C and the period of incubation is from 24 to 48 hours. In yet another embodiment, the incubation temperature is from 30°C to 37°C and the period of incubation is from 24 to 48 hours. Exemplary incubation times and temperatures include, e.g., 30 degrees Celsius overnight for *E. coli,* and 30 degrees Celsius for 48 hours for *P. pastoris.*

"Picking colonies" refers to the transfer of some or all of the cells of a colony, preferably a single colony, to fresh solid or liquid culture medium for propagation or into a mix of culture medium and glycerol for storage. This is typically achieved by scraping with a loop or dipping with a stick or pipette tip. In the method of the present invention the colonies are picked using a robot which identifies the colonies, picks them and transfers them to new medium. Robots for picking colonies are available from different companies and include the RapidPick™ Automated Colony Picking Systems of Hudson Robotics, the Stinger of Singer Instruments, the Pickolo™ Colony-Picker of Scirobotics and the Qpix 400 Series Microbial Colony Picker of Molecular Devices

The phrase "nucleic acid" refers to oligonucleotides, nucleotides, polynucleotides, or fragments of any of these, DNA or RNA (e.g., mRNA, rRNA, tRNA) of genomic, recombinant or synthetic origin which may be single-stranded or double-stranded and may represent a sense or antisense strand, peptide nucleic acid (PNA), or any DNA-like or RNA-like material, natural or synthetic in origin, including, e.g., iRNA such as miRNA or siRNA, ribonucleoproteins (e.g., iRNPs). The term encompasses nucleic acids, i.e., oligonucleotides, containing known analogues of natural nucleotides. The term "nucleic acid sequence" refers to the sequence of nucleotides within a nucleic acid molecule which defines the amino acid sequence of the encoded protein.

"Isolating DNA" refers to rupturing the cell or cells of the library members, e.g. enzymatically, mechanically, or osmotically, to release the cellular contents, and removing insoluble matter, lipids, proteins, RNA, and other non-DNA molecules using standard methods or kits available in the art.

"Sequencing DNA" refers to determining the nucleic acid sequence of a piece of DNA, e.g. of a gene. Standard methods and commercial services are known in the art. Basic methods for DNA sequencing include the Maxam-Gilbert method and the chain termination method. High-throughput techniques have also been developed and are preferably used in the method of the present invention. These high-throughput techniques include, but are not limited to, Massively parallel signature sequencing (MPSS), Polony sequencing, 454 pyrosequencing, Illumina (Solexa) sequencing, Combinatorial probe anchor synthesis (cPAS), SOLiD sequencing, Ion Torrent semiconductor sequencing, DNA nanoball sequencing, Heliscope single molecule sequencing, Single molecule real time (SMRT) sequencing and Nanopore DNA sequencing.

The method of the present invention may comprise further steps after the DNA has been sequenced. For example, the DNA sequence of a variant of a parent protein may be compared with the DNA sequence of said parent protein.

The protein of interest may have a desired function selected from the group consisting of an enzymatic activity, substrate specificity, enzyme stability, and expression titer. Enzymatic activity may comprise an activity at increased temperatures, such as temperatures of 70°C, 75° C, 80°C or 85°C or at low pH, such as a pH of 5.5, 5.0, 4.5 or 4.0. Enzymatic activity may also comprise an activity of the enzyme which is not inhibited by feedback inhibition. Substrate specificity may comprise a specificity for one specific substrate so that no byproducts are produced by reaction with other substrates. Enzyme stability may comprise stability of the enzyme at high temperatures, e.g. temperatures of 70°C, 71°C, 72°C, 73°C, 74°C, 75°C, 76°C, 77°C, 78°C, 79°C, 80°C, 81°C, 82°C, 83°C, 84°C, or 85°C or at low pH, e.g. a pH of 5.5, 5.4, 5.3, 5.2, 5.1,5.0, 4.9, 4.8, 4.7, 4.6, 4.5, 4.4, 4.3, 4.2, 4.1, or 4.0. Expression titer means that the protein of interest is expressed at a high level.

Where it is specified that a method of the invention comprises certain steps "in sequence", other steps may of course also be comprised in such a method, either before, between, or after the listed steps. The listed sequence only relates to the listed steps in relation to each other, and not in relation to any additional steps.

In an aspect, the invention provides a method for analyzing a library comprising, in sequence, the steps of:
(a) providing a library comprising a plurality of members, wherein each member comprises one or more cells, and wherein each member recombinantly expresses a protein;
(b) arraying solutions of the members of the library in one or more microtiter plates;
(c) transferring a specified volume of each solution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the cells present in the picked colonies to multiply;
(h) isolating DNA from the cells in the liquid medium; and,
(i) sequencing the DNA encoding the protein of interest.

In an embodiment, the invention provides a method for analyzing a library comprising, in sequence, the steps of:
(a) providing a library comprising a plurality of members, wherein each member comprises one or more cells, and wherein each member recombinantly expresses an enzyme, a peptide, an antibody or antigen-binding fragment thereof, a protein antibiotic, a fusion protein, a vaccine or a vaccine-like protein or particle, a growth factor, a hormone, or a cytokine;
(b) arraying solutions of the members of the library in one or more microtiter plates;
(c) transferring a specified volume of each solution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the cells present in the picked colonies to multiply;
(h) isolating DNA from the cells in the liquid medium; and,
(i) sequencing the DNA encoding the protein of interest.

In one such embodiment, the invention provides a method for analyzing a library comprising, in sequence, the steps of:
(a) providing a library comprising a plurality of members, wherein each member comprises one or more cells, and wherein each member recombinantly expresses an enzyme selected from the group consisting of phytase, protease, beta-glucanase, xylanase, mannanase, lipase, cellulase, glucoamylase, amylase, alpha-amylase, and beta-amylase;
(b) arraying solutions of the members of the library in one or more microtiter plates;
(c) transferring a specified volume of each solution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the cells present in the picked colonies to multiply;
(h) isolating DNA from the cells in the liquid medium; and,
(i) sequencing the DNA encoding the enzyme.

In an embodiment, in any of the preceding methods, the one or more cells may be selected from the group consisting of prokaryotic cells and eukaryotic cells. That is, each member of the library to be analyzed comprises one or more prokaryotic cells or eukaryotic cells. In one such embodiment, the one or more cells are prokaryotic cells and the prokaryotic cells are bacterial cells, i.e. each member of the library to be analyzed comprises one or more bacterial cells. In another such embodiment, the one or more cells are eukaryotic cells, and the eukaryotic cells are selected from the group consisting of fungal cells, yeast cells, animal cells, and plant cells, i.e. each member of the library to be analyzed comprises one or more fungal cells, yeast cells, animal cells, or plant cells.

In another embodiment, the one or more cells are selected from the group consisting of *E. coli* cells, *Bacillus* cells, *Trichoderma* cells, *Komagataella* cells, *Aspergillus* cells, *Thermothelomyces* cells, and *Saccharomyces* cells. Accordingly the invention provides a method for analyzing a library comprising, in sequence, the steps of:
(a) providing a library comprising a plurality of members, wherein each member comprises one or more *E. coli* cells, *Bacillus* cells, *Trichoderma* cells, *Komagataella* cells, *Aspergillus* cells, *Thermothelomyces* cells, or *Saccharomyces* cells, and wherein each member recombinantly expresses a protein;
(b) arraying solutions of the members of the library in one or more microtiter plates;
(c) transferring a specified volume of each solution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the *E*. *coli* cells, *Bacillus* cells, *Trichoderma* cells, *Komagataella* cells, *Aspergillus* cells, *Thermothelomyces* cells, or *Saccharomyces* cells present in the picked colonies to multiply;
(h) isolating DNA from the *E. coli* cells*, Bacillus* cells, *Trichoderma* cells, *Komagataella* cells, *Aspergillus* cells, *Thermothelomyces* cells, or *Saccharomyces* cells in the liquid medium; and,
(i) sequencing the DNA encoding the protein of interest.

Further, the invention provides a method for analyzing a library comprising, in sequence, the steps of:
(a) providing a library comprising a plurality of members, wherein each member comprises one or more *E. coli* cells*, Bacillus* cells, *Trichoderma* cells, *Komagataella* cells, *Aspergillus* cells, *Thermothelomyces* cells, or *Saccharomyces* cells, and wherein each member recombinantly expresses an enzyme selected from the group consisting of phytase, protease, beta-glucanase, xylanase, mannanase, lipase, cellulase, glucoamylase, amylase, alpha-amylase, and beta-amylase;
(b) arraying solutions of the members of the library in one or more microtiter plates;
(c) transferring a specified volume of each solution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the *E*. *coli* cells, *Bacillus* cells, *Trichoderma* cells, *Komagataella* cells, *Aspergillus* cells, *Thermothelomyces* cells, or *Saccharomyces* cells present in the picked colonies to multiply;
(h) isolating DNA from the *E. coli* cells, *Bacillus* cells, *Trichoderma* cells, *Komagataella* cells, *Aspergillus* cells, *Thermothelomyces* cells, or *Saccharomyces* cells in the liquid medium; and,
(i) sequencing the DNA encoding the enzyme.

Further, the invention provides a method for analyzing a library comprising, in sequence, the steps of:
(a) providing a library comprising a plurality of members, wherein each member comprises one or more *E. coli* cells and wherein each member recombinantly expresses an enzyme selected from the group consisting of phytase, protease, beta-glucanase, xylanase, mannanase, lipase, cellulase, glucoamylase, amylase, alpha-amylase, and beta-amylase;
(b) arraying solutions of the members of the library in one or more microtiter plates;
(c) transferring a specified volume of each solution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the *E*. *coli* cells present in the picked colonies to multiply;
(h) isolating DNA from the *E. coli* cells in the liquid medium; and,
(i) sequencing the DNA encoding the enzyme.

Further, the invention provides a method for analyzing a library comprising, in sequence, the steps of:
(a) providing a library comprising a plurality of members, wherein each member comprises one or more *Komagataella phaffii* cells, and wherein each member recombinantly expresses an enzyme selected from the group consisting of phytase, protease, beta-glucanase, xylanase, mannanase, lipase, cellulase, glucoamylase, amylase, alpha-amylase, and beta-amylase;
(b) arraying solutions of the members of the library in one or more microtiter plates;
(c) transferring a specified volume of each solution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the *Komagataella phaffii* cells present in the picked colonies to multiply;
(h) isolating DNA from the *Komagataella phaffii* cells in the liquid medium; and,
(i) sequencing the DNA encoding the enzyme.

Further, the invention provides a method for analyzing a library comprising, in sequence, the steps of:
(a) providing a library comprising a plurality of members, wherein each member comprises one or more *Bacillus* cells, and wherein each member recombinantly expresses an enzyme selected from the group consisting of phytase, protease, beta-glucanase, xylanase, mannanase, lipase, cellulase, glucoamylase, amylase, alpha-amylase, and beta-amylase;
(b) arraying solutions of the members of the library in one or more microtiter plates;
(c) transferring a specified volume of each solution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the *Bacillus* cells present in the picked colonies to multiply;
(h) isolating DNA from the *Bacillus* cells in the liquid medium; and,
(i) sequencing the DNA encoding the enzyme.

In another embodiment, in any of the preceding methods and embodiments, the protein has a desired function selected from the group consisting of an enzymatic activity, a substrate specificity, an enzymatic stability, an expression titer, and any combination thereof.

In yet another embodiment, the microtiter plate comprises wells. Accordingly, the invention provides a method for analyzing a library comprising, in sequence, the steps of:
(a) providing a library comprising a plurality of members, wherein each member comprises one or more cells, and wherein each member recombinantly expresses a protein;
(b) arraying solutions of the members of the library in one or more microtiter plates comprising wells;
(c) transferring a specified volume of each solution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the cells present in the picked colonies to multiply;
(h) isolating DNA from the cells in the liquid medium; and,
(i) sequencing the DNA encoding the protein of interest.

Further, the invention provides a method for analyzing a library comprising, in sequence, the steps of:
(a) providing a library comprising a plurality of members, wherein each member comprises one or more cells, and wherein each member recombinantly expresses an enzyme, a peptide, an antibody or antigen-binding fragment thereof, a protein antibiotic, a fusion protein, a vaccine or a vaccine-like protein or particle, a growth factor, a hormone, or a cytokine;
(b) arraying solutions of the members of the library in one or more microtiter plates comprising wells;
(c) transferring a specified volume of each solution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the cells present in the picked colonies to multiply;
(h) isolating DNA from the cells in the liquid medium; and,
(i) sequencing the DNA encoding the protein of interest.

Further, the invention provides a method for analyzing a library comprising, in sequence, the steps of:
(a) providing a library comprising a plurality of members, wherein each member comprises one or more cells, and wherein each member recombinantly expresses an enzyme selected from the group consisting of phytase, protease, beta-glucanase, xylanase, mannanase, lipase, cellulase, glucoamylase, amylase, alpha-amylase, and beta-amylase;
(b) arraying solutions of the members of the library in one or more microtiter plates comprising wells;
(c) transferring a specified volume of each solution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the cells present in the picked colonies to multiply;
(h) isolating DNA from the cells in the liquid medium; and,
(i) sequencing the DNA encoding the enzyme.

In an embodiment, in any of the preceding methods in which the one or more microtiter plates comprise wells, the one or more cells may be selected from the group consisting of prokaryotic cells and eukaryotic cells. That is, each member of the library to be analyzed comprises one or more prokaryotic cells or eukaryotic cells. In one such embodiment, the one or more cells are prokaryotic cells and the prokaryotic cells are bacterial cells, i.e. each member of the library to be analyzed comprises one or more bacterial cells. In another such embodiment, the one or more cells are eukaryotic cells, and the eukaryotic cells are selected from the group consisting of fungal cells, yeast cells, animal cells, and plant cells, i.e. each member of the library to be analyzed comprises one or more fungal cells, yeast cells, animal cells, or plant cells.

Further, the invention provides a method for analyzing a library comprising, in sequence, the steps of:
(a) providing a library comprising a plurality of members, wherein each member comprises one or more *E. coli* cells*, Bacillus* cells, *Trichoderma* cells, *Komagataella* cells, *Aspergillus* cells, *Thermothelomyces* cells, or *Saccharomyces* cells, and wherein each member recombinantly expresses a protein;
(b) arraying solutions of the members of the library in one or more microtiter plates comprising wells;
(c) transferring a specified volume of each solution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the *E*. *coli* cells, *Bacillus* cells, *Trichoderma* cells, *Komagataella* cells, *Aspergillus* cells, *Thermothelomyces* cells, or *Saccharomyces* cells present in the picked colonies to multiply;
(h) isolating DNA from the *E. coli* cells, *Bacillus* cells, *Trichoderma* cells, *Komagataella* cells, *Aspergillus* cells, *Thermothelomyces* cells, or *Saccharomyces* cells in the liquid medium; and,
(i) sequencing the DNA encoding the protein of interest.

Further, the invention provides a method for analyzing a library comprising, in sequence, the steps of:
(a) providing a library comprising a plurality of members, wherein each member comprises one or more *E. coli* cells, *Bacillus* cells, *Trichoderma* cells, *Komagataella* cells, *Aspergillus* cells, *Thermothelomyces* cells, or *Saccharomyces* cells, and wherein each member recombinantly expresses an enzyme, a peptide, an antibody or antigen-binding fragment thereof, a protein antibiotic, a fusion protein, a vaccine or a vaccine-like protein or particle, a growth factor, a hormone, or a cytokine;
(b) arraying solutions of the members of the library in one or more microtiter plates comprising wells;
(c) transferring a specified volume of each solution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the *E*. *coli* cells*, Bacillus* cells, *Trichoderma* cells, *Komagataella* cells, *Aspergillus* cells, *Thermothelomyces* cells, or *Saccharomyces* cells present in the picked colonies to multiply;
(h) isolating DNA from the *E. coli* cells, *Bacillus* cells, *Trichoderma* cells, *Komagataella* cells, *Aspergillus* cells, *Thermothelomyces* cells, or *Saccharomyces* cells in the liquid medium; and,
(i) sequencing the DNA encoding the protein of interest.

Further, the invention provides a method for analyzing a library comprising, in sequence, the steps of:
(a) providing a library comprising a plurality of members, wherein each member comprises one or more *E. coli* cells, *Bacillus* cells, *Trichoderma* cells, *Komagataella* cells, *Aspergillus* cells, *Thermothelomyces* cells, or *Saccharomyces* cells, and wherein each member recombinantly expresses an enzyme selected from the group consisting of phytase, protease, beta-glucanase, xylanase, mannanase, lipase, cellulase, glucoamylase, amylase, alpha-amylase, and beta-amylase;
(b) arraying solutions of the members of the library in one or more microtiter plates comprising wells;
(c) transferring a specified volume of each solution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the *E*. *coli* cells, *Bacillus* cells, *Trichoderma* cells, *Komagataella* cells, *Aspergillus* cells, *Thermothelomyces* cells, or *Saccharomyces* cells present in the picked colonies to multiply;
(h) isolating DNA from the *E. coli* cells, *Bacillus* cells, *Trichoderma* cells, *Komagataella* cells, *Aspergillus* cells, *Thermothelomyces* cells, or *Saccharomyces* cells in the liquid medium; and,
(i) sequencing the DNA encoding the enzyme.

Further, the invention provides a method for analyzing a library comprising, in sequence, the steps of:
(a) providing a library comprising a plurality of members, wherein each member comprises one or more *E. coli* cells*,* and wherein each member recombinantly expresses an enzyme selected from the group consisting of phytase, protease, beta-glucanase, xylanase, mannanase, lipase, cellulase, glucoamylase, amylase, alpha-amylase, and beta-amylase;
(b) arraying solutions of the members of the library in one or more microtiter plates comprising wells;
(c) transferring a specified volume of each solution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the *E*. *coli* cells present in the picked colonies to multiply;
(h) isolating DNA from the *E. coli* cells in the liquid medium; and
(i) sequencing the DNA encoding the enzyme.

Further, the invention provides a method for analyzing a library comprising, in sequence, the steps of:
(a) providing a library comprising a plurality of members, wherein each member comprises one or more *Komagataella phaffii* cells, and wherein each member recombinantly expresses an enzyme selected from the group consisting of phytase, protease, beta-glucanase, xylanase, mannanase, lipase, cellulase, glucoamylase, amylase, alpha-amylase, and beta-amylase;
(b) arraying solutions of the members of the library in one or more microtiter plates comprising wells;
(c) transferring a specified volume of each solution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the *Komagataella phaffii* cells present in the picked colonies to multiply;
(h) isolating DNA from the *Komagataella phaffii* cells in the liquid medium; and
(i) sequencing the DNA encoding the enzyme.

In an embodiment of any of the preceding methods and embodiments in which the one or more microtiter plates comprise wells, the protein has a desired function selected from the group consisting of an enzymatic activity, a substrate specificity, an enzymatic stability, an expression titer, and any combination thereof.

The number of wells in the one or more microtiter plates of any of the above methods or embodiments in which the one or more microtiter plates comprise wells may be, e.g., 6, 12, 24, 48, 96, 384, or 1536.

In an embodiment, in step (b) of any one of the inventive methods and embodiments, one or more dilutions of the solutions are prepared before the members are arrayed, optionally wherein the dilutions are serial dilutions. In one embodiment, the one or more dilutions are arrayed together with the undiluted solutions. Dilutions can be made in liquid growth media, buffers, or ultrapure water (e.g. Milli-Q water). Such media, buffers, and ultrapure water are known in the art. In one embodiment, one to ten dilutions of the solutions are prepared, preferably two to seven dilutions of the solutions are prepared and more preferably three to five dilutions of the solutions are prepared. A suitable dilution factor is 1:10, so that the first dilution is 1:10, the second dilution is 1:100, the third solution is 1:1,000 and so on. The dilution preferably leads to the formation of single colonies on the agar trays so that single members can be isolated without contamination by other members.

Accordingly, the invention provides a method for analyzing a library comprising, in sequence, the steps of:
(a) providing a library comprising a plurality of members, wherein each member comprises one or more cells, and wherein each member recombinantly expresses a protein;
(b) preparing dilutions of solutions of the members of the library and arraying the solutions and dilutions in one or more microtiter plates, optionally comprising wells;
(c) transferring a specified volume of each solution and dilution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the cells present in the picked colonies to multiply;
(h) isolating DNA from the cells in the liquid medium; and,
(i) sequencing the DNA encoding the protein of interest.

Further, the invention provides a method for analyzing a library comprising, in sequence, the steps of:
(a) providing a library comprising a plurality of members, wherein each member comprises one or more cells, and wherein each member recombinantly expresses a protein;
(b) preparing serial dilutions of solutions of the members of the library and arraying the solutions and serial dilutions in one or more microtiter plates, optionally comprising wells;
(c) transferring a specified volume of each solution and serial dilution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the cells present in the picked colonies to multiply;
(h) isolating DNA from the cells in the liquid medium; and,
(i) sequencing the DNA encoding the protein of interest.

Further, the invention provides a method for analyzing a library comprising, in sequence, the steps of:
(a) providing a library comprising a plurality of members, wherein each member comprises one or more cells, and wherein each member recombinantly expresses an enzyme, a peptide, an antibody or antigen-binding fragment thereof, a protein antibiotic, a fusion protein, a vaccine or a vaccine-like protein or particle, a growth factor, a hormone, or a cytokine;
(b) preparing dilutions of solutions of the members of the library and arraying the solutions and dilutions in one or more microtiter plates, optionally comprising wells;
(c) transferring a specified volume of each solution and dilution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the cells present in the picked colonies to multiply;
(h) isolating DNA from the cells in the liquid medium; and,
(i) sequencing the DNA encoding the protein of interest.

Further, the invention provides a method for analyzing a library comprising, in sequence, the steps of:
(a) providing a library comprising a plurality of members, wherein each member comprises one or more cells, and wherein each member recombinantly expresses an enzyme, a peptide, an antibody or antigen-binding fragment thereof, a protein antibiotic, a fusion protein, a vaccine or a vaccine-like protein or particle, a growth factor, a hormone, or a cytokine;
(b) preparing serial dilutions of solutions of the members of the library and arraying the solutions and serial dilutions in one or more microtiter plates, optionally comprising wells;
(c) transferring a specified volume of each solution and serial dilution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the cells present in the picked colonies to multiply;
(h) isolating DNA from the cells in the liquid medium; and,
(i) sequencing the DNA encoding the protein of interest.

Further, the invention provides a method for analyzing a library comprising, in sequence, the steps of:
(a) providing a library comprising a plurality of members, wherein each member comprises one or more cells, and wherein each member recombinantly expresses an enzyme selected from the group consisting of phytase, protease, beta-glucanase, xylanase, mannanase, lipase, cellulase, glucoamylase, amylase, alpha-amylase, and beta-amylase;
(b) preparing dilutions of solutions of the members of the library and arraying the solutions and dilutions in one or more microtiter plates, optionally comprising wells;
(c) transferring a specified volume of each solution and dilution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the cells present in the picked colonies to multiply;
(h) isolating DNA from the cells in the liquid medium; and,
(i) sequencing the DNA encoding the enzyme.

Further, the invention provides a method for analyzing a library comprising, in sequence, the steps of:
(a) providing a library comprising a plurality of members, wherein each member comprises one or more cells, and wherein each member recombinantly expresses an enzyme selected from the group consisting of phytase, protease, beta-glucanase, xylanase, mannanase, lipase, cellulase, glucoamylase, amylase, alpha-amylase, and beta-amylase;
(b) preparing serial dilutions of solutions of the members of the library and arraying the solutions and serial dilutions in one or more microtiter plates, optionally comprising wells;
(c) transferring a specified volume of each solution and serial dilution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the cells present in the picked colonies to multiply;
(h) isolating DNA from the cells in the liquid medium; and,
(i) sequencing the DNA encoding the enzyme.

In an embodiment, in any of the preceding methods in which dilutions or serial dilutions are performed in step (b), the one or more cells may be selected from the group consisting of prokaryotic cells and eukaryotic cells. That is, each member of the library to be analyzed comprises one or more prokaryotic cells or eukaryotic cells. In one such embodiment, the one or more cells are prokaryotic cells and the prokaryotic cells are bacterial cells, i.e. each member of the library to be analyzed comprises one or more bacterial cells. In another such embodiment, the one or more cells are eukaryotic cells, and the eukaryotic cells are selected from the group consisting of fungal cells, yeast cells, animal cells, and plant cells, i.e. each member of the library to be analyzed comprises one or more fungal cells, yeast cells, animal cells, or plant cells.

Further, the invention provides a method for analyzing a library comprising, in sequence, the steps of:
(a) providing a library comprising a plurality of members, wherein each member comprises one or more *E. coli* cells*, Bacillus* cells, *Trichoderma* cells, *Komagataella* cells, *Aspergillus* cells, *Thermothelomyces* cells, or *Saccharomyces* cells, and wherein each member recombinantly expresses a protein;
(b) preparing dilutions of solutions of the members of the library and arraying the solutions and dilutions in one or more microtiter plates, optionally comprising wells;
(c) transferring a specified volume of each solution and dilution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the *E*. *coli* cells, *Bacillus* cells, *Trichoderma* cells, *Komagataella* cells, *Aspergillus* cells, *Thermothelomyces* cells, or *Saccharomyces* cells present in the picked colonies to multiply;
(h) isolating DNA from the *E. coli* cells, *Bacillus* cells, *Trichoderma* cells, *Komagataella* cells, *Aspergillus* cells, *Thermothelomyces* cells, or *Saccharomyces* cells in the liquid medium; and,
(i) sequencing the DNA encoding the protein of interest.

Further, the invention provides a method for analyzing a library comprising, in sequence, the steps of:
(a) providing a library comprising a plurality of members, wherein each member comprises one or more *E. coli* cells, *Bacillus* cells, *Trichoderma* cells, *Komagataella* cells, *Aspergillus* cells, *Thermothelomyces* cells, or *Saccharomyces* cells, and wherein each member recombinantly expresses a protein;
(b) preparing serial dilutions of solutions of the members of the library and arraying the solutions and serial dilutions in one or more microtiter plates, optionally comprising wells;
(c) transferring a specified volume of each solution and serial dilution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the *E*. *coli* cells*, Bacillus* cells, *Trichoderma* cells, *Komagataella* cells, *Aspergillus* cells, *Thermothelomyces* cells, or *Saccharomyces* cells present in the picked colonies to multiply;
(h) isolating DNA from the *E. coli* cells, *Bacillus* cells, *Trichoderma* cells, *Komagataella* cells, *Aspergillus* cells, *Thermothelomyces* cells, or *Saccharomyces* cells in the liquid medium; and,
(i) sequencing the DNA encoding the protein of interest.

Further, the invention provides a method for analyzing a library comprising, in sequence, the steps of:
(a) providing a library comprising a plurality of members, wherein each member comprises one or more *E. coli* cells*, Bacillus* cells, *Trichoderma* cells, *Komagataella* cells, *Aspergillus* cells, *Thermothelomyces* cells, or *Saccharomyces* cells, and wherein each member recombinantly expresses an enzyme, a peptide, an antibody or antigen-binding fragment thereof, a protein antibiotic, a fusion protein, a vaccine or a vaccine-like protein or particle, a growth factor, a hormone, or a cytokine;
(b) preparing dilutions of solutions of the members of the library and arraying the solutions and dilutions in one or more microtiter plates, optionally comprising wells;
(c) transferring a specified volume of each solution and dilution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the *E*. *coli* cells, *Bacillus* cells, *Trichoderma* cells, *Komagataella* cells, *Aspergillus* cells, *Thermothelomyces* cells, or *Saccharomyces* cells present in the picked colonies to multiply;
(h) isolating DNA from the *E. coli* cells, *Bacillus* cells, *Trichoderma* cells, *Komagataella* cells, *Aspergillus* cells, *Thermothelomyces* cells, or *Saccharomyces* cells in the liquid medium; and,
(i) sequencing the DNA encoding the protein of interest.

Further, the invention provides a method for analyzing a library comprising, in sequence, the steps of:
(a) providing a library comprising a plurality of members, wherein each member comprises one or more *E. coli* cells, *Bacillus* cells, *Trichoderma* cells, *Komagataella* cells, *Aspergillus* cells, *Thermothelomyces* cells, or *Saccharomyces* cells, and wherein each member recombinantly expresses an enzyme, a peptide, an antibody or antigen-binding fragment thereof, a protein antibiotic, a fusion protein, a vaccine or a vaccine-like protein or particle, a growth factor, a hormone, or a cytokine;
(b) preparing serial dilutions of solutions of the members of the library and arraying the solutions and serial dilutions in one or more microtiter plates, optionally comprising wells;
(c) transferring a specified volume of each solution and serial dilution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the *E*. *coli* cells*, Bacillus* cells, *Trichoderma* cells, *Komagataella* cells, *Aspergillus* cells, *Thermothelomyces* cells, or *Saccharomyces* cells present in the picked colonies to multiply;
(h) isolating DNA from the *E. coli* cells, *Bacillus* cells, *Trichoderma* cells, *Komagataella* cells, *Aspergillus* cells, *Thermothelomyces* cells, or *Saccharomyces* cells in the liquid medium; and,
(i) sequencing the DNA encoding the protein of interest.

Further, the invention provides a method for analyzing a library comprising, in sequence, the steps of:
(a) providing a library comprising a plurality of members, wherein each member comprises one or more *E. coli* cells*, Bacillus* cells, *Trichoderma* cells, *Komagataella* cells, *Aspergillus* cells, *Thermothelomyces* cells, or *Saccharomyces* cells, and wherein each member recombinantly expresses an enzyme selected from the group consisting of phytase, protease, beta-glucanase, xylanase, mannanase, lipase, cellulase, glucoamylase, amylase, alpha-amylase, and beta-amylase;
(b) preparing dilutions of solutions of the members of the library and arraying the solutions and dilutions in one or more microtiter plates, optionally comprising wells;
(c) transferring a specified volume of each solution and dilution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the *E*. *coli* cells, *Bacillus* cells, *Trichoderma* cells, *Komagataella* cells, *Aspergillus* cells, *Thermothelomyces* cells, or *Saccharomyces* cells present in the picked colonies to multiply;
(h) isolating DNA from the *E. coli* cells, *Bacillus* cells, *Trichoderma* cells, *Komagataella* cells, *Aspergillus* cells, *Thermothelomyces* cells, or *Saccharomyces* cells in the liquid medium; and,
(i) sequencing the DNA encoding the enzyme.

Further, the invention provides a method for analyzing a library comprising, in sequence, the steps of:
(a) providing a library comprising a plurality of members, wherein each member comprises one or more *E. coli* cells, and wherein each member recombinantly expresses an enzyme selected from the group consisting of phytase, protease, beta-glucanase, xylanase, mannanase, lipase, cellulase, glucoamylase, amylase, alpha-amylase, and beta-amylase;
(b) preparing dilutions of solutions of the members of the library and arraying the solutions and dilutions in one or more microtiter plates, optionally comprising wells;
(c) transferring a specified volume of each solution and dilution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the *E*. *coli* cells present in the picked colonies to multiply;
(h) isolating DNA from the *E. coli* cells in the liquid medium; and,
(i) sequencing the DNA encoding the enzyme.

Further, the invention provides a method for analyzing a library comprising, in sequence, the steps of:
(a) providing a library comprising a plurality of members, wherein each member comprises one or more *Komagataella phaffii* cells, and wherein each member recombinantly expresses an enzyme selected from the group consisting of phytase, protease, beta-glucanase, xylanase, mannanase, lipase, cellulase, glucoamylase, amylase, alpha-amylase, and beta-amylase;
(b) preparing dilutions of solutions of the members of the library and arraying the solutions and dilutions in one or more microtiter plates, optionally comprising wells;
(c) transferring a specified volume of each solution and dilution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the *Komagataella phaffii* cells present in the picked colonies to multiply;
(h) isolating DNA from the *Komagataella phaffii* cells in the liquid medium; and
(i) sequencing the DNA encoding the enzyme.

Further, the invention provides a method for analyzing a library comprising, in sequence, the steps of:
(a) providing a library comprising a plurality of members, wherein each member comprises one or more *E. coli* cells*, Bacillus* cells, *Trichoderma* cells, *Komagataella* cells, *Aspergillus* cells, *Thermothelomyces* cells, or *Saccharomyces* cells, and wherein each member recombinantly expresses an enzyme selected from the group consisting of phytase, protease, beta-glucanase, xylanase, mannanase, lipase, cellulase, glucoamylase, amylase, alpha-amylase, and beta-amylase;
(b) preparing serial dilutions of solutions of the members of the library and arraying the solutions and serial dilutions in one or more microtiter plates, optionally comprising wells;
(c) transferring a specified volume of each solution and serial dilution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the *E*. *coli* cells*, Bacillus* cells, *Trichoderma* cells, *Komagataella* cells, *Aspergillus* cells, *Thermothelomyces* cells, or *Saccharomyces* cells present in the picked colonies to multiply;
(h) isolating DNA from the *E. coli* cells, *Bacillus* cells, *Trichoderma* cells, *Komagataella* cells, *Aspergillus* cells, *Thermothelomyces* cells, or *Saccharomyces* cells in the liquid medium; and,
(i) sequencing the DNA encoding the enzyme.

Further, the invention provides a method for analyzing a library comprising, in sequence, the steps of:
(a) providing a library comprising a plurality of members, wherein each member comprises one or more *E. coli* cells*,* and wherein each member recombinantly expresses an enzyme selected from the group consisting of phytase, protease, beta-glucanase, xylanase, mannanase, lipase, cellulase, glucoamylase, amylase, alpha-amylase, and beta-amylase;
(b) preparing serial dilutions of solutions of the members of the library and arraying the solutions and serial dilutions in one or more microtiter plates, optionally comprising wells;
(c) transferring a specified volume of each solution and serial dilution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the *E*. *coli* cells present in the picked colonies to multiply;
(h) isolating DNA from the *E. coli* cells in the liquid medium; and
(i) sequencing the DNA encoding the enzyme.

Further, the invention provides a method for analyzing a library comprising, in sequence, the steps of:
(a) providing a library comprising a plurality of members, wherein each member comprises one or more *Komagataella phaffii* cells, and wherein each member recombinantly expresses an enzyme selected from the group consisting of phytase, protease, beta-glucanase, xylanase, mannanase, lipase, cellulase, glucoamylase, amylase, alpha-amylase, and beta-amylase;
(b) preparing serial dilutions of solutions of the members of the library and arraying the solutions and serial dilutions in one or more microtiter plates, optionally comprising wells;
(c) transferring a specified volume of each solution and serial dilution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the *Komagataella phaffii* cells present in the picked colonies to multiply;
(h) isolating DNA from the *Komagataella phaffii* cells in the liquid medium; and
(i) sequencing the DNA encoding the enzyme.

The number of wells in the one or more microtiter plates of any of the above methods or embodiments in which the one or more microtiter plates comprise wells may be, e.g., 6, 12, 24, 48, 96, 384, or 1536.

In an embodiment, in any of the preceding methods and embodiments in which dilutions or serial dilutions are performed in step (b) and the one or more microtiter plates comprise wells, the protein has a desired function selected from the group consisting of an enzymatic activity, a substrate specificity, an enzymatic stability, an expression titer, and any combination thereof.

In an embodiment, in any of the above methods or embodiments, the solutions and/or dilutions of the solutions comprise a liquid comprising a glycerol stock. The glycerol stock may comprise, e.g. 10%-60%, preferably 17-30%, by volume of glycerol.

In an embodiment, in any of the above methods and embodiments, the specified volume transferred from the one or more microtiter plates onto the one or more agar trays using the acoustic dispenser in step (c) of the inventive method is 1 nL, 2 nL, 3 nL, 4 nL, 5 nL, 6 nL, 7 nL, 8 nL,9 nL, 10 nL, 11 nL, 12 nL, 13 nL, 14 nL, 15 nL,16 nL, 17 nL, 18 nL, 19 nL, 20 nL, 21 nL, 22 nL, 23 nL, 24 nL, 25 nL, 26 nL, 27 nL, 28 nL, 29 nL, or 30 nL.

That is, the invention provides a method for analyzing a library comprising, in sequence, the steps of:
(a) providing a library comprising a plurality of members, wherein each member comprises one or more cells, and wherein each member recombinantly expresses a protein;
(b) arraying solutions of the members of the library in one or more microtiter plates;
(c) transferring a specified volume being 1 nL, 2 nL, 3 nL, 4 nL, 5 nL, 6 nL, 7 nL, 8 nL,9 nL, 10 nL, 11 nL, 12 nL, 13 nL, 14 nL, 15 nL,16 nL, 17 nL, 18 nL, 19 nL, 20 nL, 21 nL, 22 nL, 23 nL, 24 nL, 25 nL, 26 nL, 27 nL, 28 nL, 29 nL, or 30 nL of each solution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the cells present in the picked colonies to multiply;
(h) isolating DNA from the cells in the liquid medium; and,
(i) sequencing the DNA encoding the protein of interest.

Further, the invention provides a method for analyzing a library comprising, in sequence, the steps of:
(a) providing a library comprising a plurality of members, wherein each member comprises one or more cells, and wherein each member recombinantly expresses an enzyme, a peptide, an antibody or antigen-binding fragment thereof, a protein antibiotic, a fusion protein, a vaccine or a vaccine-like protein or particle, a growth factor, a hormone, or a cytokine;
(b) arraying solutions of the members of the library in one or more microtiter plates;
(c) transferring a specified volume being 1 nL, 2 nL, 3 nL, 4 nL, 5 nL, 6 nL, 7 nL, 8 nL,9 nL, 10 nL, 11 nL, 12 nL, 13 nL, 14 nL, 15 nL,16 nL, 17 nL, 18 nL, 19 nL, 20 nL, 21 nL, 22 nL, 23 nL, 24 nL, 25 nL, 26 nL, 27 nL, 28 nL, 29 nL, or 30 nL of each solution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the cells present in the picked colonies to multiply;
(h) isolating DNA from the cells in the liquid medium; and,
(i) sequencing the DNA encoding the protein of interest.

Further, the invention provides a method for analyzing a library comprising, in sequence, the steps of:
(a) providing a library comprising a plurality of members, wherein each member comprises one or more cells, and wherein each member recombinantly expresses an enzyme selected from the group consisting of phytase, protease, beta-glucanase, xylanase, mannanase, lipase, cellulase, glucoamylase, amylase, alpha-amylase, and beta-amylase;
(b) arraying solutions of the members of the library in one or more microtiter plates;
(c) transferring a specified volume being 1 nL, 2 nL, 3 nL, 4 nL, 5 nL, 6 nL, 7 nL, 8 nL,9 nL, 10 nL, 11 nL, 12 nL, 13 nL, 14 nL, 15 nL,16 nL, 17 nL, 18 nL, 19 nL, 20 nL, 21 nL, 22 nL, 23 nL, 24 nL, 25 nL, 26 nL, 27 nL, 28 nL, 29 nL, or 30 nL of each solution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the cells present in the picked colonies to multiply;
(h) isolating DNA from the cells in the liquid medium; and,
(i) sequencing the DNA encoding the enzyme.

In an embodiment, in any of the preceding methods in which a specified volume being 1 nL, 2 nL, 3 nL, 4 nL, 5 nL, 6 nL, 7 nL, 8 nL,9 nL, 10 nL, 11 nL, 12 nL, 13 nL, 14 nL, 15 nL,16 nL, 17 nL, 18 nL, 19 nL, 20 nL, 21 nL, 22 nL, 23 nL, 24 nL, 25 nL, 26 nL, 27 nL, 28 nL, 29 nL, or 30 nL, is transferred in step (c), the one or more cells may be selected from the group consisting of prokaryotic cells and eukaryotic cells. That is, each member of the library to be analyzed comprises one or more prokaryotic cells or eukaryotic cells. In one such embodiment, the one or more cells are prokaryotic cells and the prokaryotic cells are bacterial cells, i.e. each member of the library to be analyzed comprises one or more bacterial cells. In another such embodiment, the one or more cells are eukaryotic cells, and the eukaryotic cells are selected from the group consisting of fungal cells, yeast cells, animal cells, and plant cells, i.e. each member of the library to be analyzed comprises one or more fungal cells, yeast cells, animal cells, or plant cells.

Further, the invention provides a method for analyzing a library comprising, in sequence, the steps of:
(a) providing a library comprising a plurality of members, wherein each member comprises one or more *E. coli* cells*, Bacillus* cells, *Trichoderma* cells, *Komagataella* cells, *Aspergillus* cells, *Thermothelomyces* cells, or *Saccharomyces* cells, and wherein each member recombinantly expresses a protein;
(b) arraying solutions of the members of the library in one or more microtiter plates;
(c) transferring a specified volume being 1 nL, 2 nL, 3 nL, 4 nL, 5 nL, 6 nL, 7 nL, 8 nL,9 nL, 10 nL, 11 nL, 12 nL, 13 nL, 14 nL, 15 nL,16 nL, 17 nL, 18 nL, 19 nL, 20 nL, 21 nL, 22 nL, 23 nL, 24 nL, 25 nL, 26 nL, 27 nL, 28 nL, 29 nL, or 30 nL of each solution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the *E*. *coli* cells*, Bacillus* cells, *Trichoderma* cells, *Komagataella* cells, *Aspergillus* cells, *Thermothelomyces* cells, or *Saccharomyces* cells present in the picked colonies to multiply;
(h) isolating DNA from the *E. coli* cells, *Bacillus* cells, *Trichoderma* cells, *Komagataella* cells, *Aspergillus* cells, *Thermothelomyces* cells, or *Saccharomyces* cells in the liquid medium; and,
(i) sequencing the DNA encoding the protein of interest.

Further, the invention provides a method for analyzing a library comprising, in sequence, the steps of:
(a) providing a library comprising a plurality of members, wherein each member comprises one or more *E. coli* cells, *Bacillus* cells, *Trichoderma* cells, *Komagataella* cells, *Aspergillus* cells, *Thermothelomyces* cells, or *Saccharomyces* cells, and wherein each member recombinantly expresses an enzyme, a peptide, an antibody or antigen-binding fragment thereof, a protein antibiotic, a fusion protein, a vaccine or a vaccine-like protein or particle, a growth factor, a hormone, or a cytokine;
(b) arraying solutions of the members of the library in one or more microtiter plates;
(c) transferring a specified volume being 1 nL, 2 nL, 3 nL, 4 nL, 5 nL, 6 nL, 7 nL, 8 nL,9 nL, 10 nL, 11 nL, 12 nL, 13 nL, 14 nL, 15 nL,16 nL, 17 nL, 18 nL, 19 nL, 20 nL, 21 nL, 22 nL, 23 nL, 24 nL, 25 nL, 26 nL, 27 nL, 28 nL, 29 nL, or 30 nL of each solution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the *E*. *coli* cells, *Bacillus* cells, *Trichoderma* cells, *Komagataella* cells, *Aspergillus* cells, *Thermothelomyces* cells, or *Saccharomyces* cells present in the picked colonies to multiply;
(h) isolating DNA from the *E. coli* cells, *Bacillus* cells, *Trichoderma* cells, *Komagataella* cells, *Aspergillus* cells, *Thermothelomyces* cells, or *Saccharomyces* cells in the liquid medium; and,
(i) sequencing the DNA encoding the protein of interest.

Further, the invention provides a method for analyzing a library comprising, in sequence, the steps of:
(a) providing a library comprising a plurality of members, wherein each member comprises one or more *E. coli* cells, *Bacillus* cells, *Trichoderma* cells, *Komagataella* cells, *Aspergillus* cells, *Thermothelomyces* cells, or *Saccharomyces* cells, and wherein each member recombinantly expresses an enzyme selected from the group consisting of phytase, protease, beta-glucanase, xylanase, mannanase, lipase, cellulase, glucoamylase, amylase, alpha-amylase, and beta-amylase;
(b) arraying solutions of the members of the library in one or more microtiter plates;
(c) transferring a specified volume being 1 nL, 2 nL, 3 nL, 4 nL, 5 nL, 6 nL, 7 nL, 8 nL,9 nL, 10 nL, 11 nL, 12 nL, 13 nL, 14 nL, 15 nL,16 nL, 17 nL, 18 nL, 19 nL, 20 nL, 21 nL, 22 nL, 23 nL, 24 nL, 25 nL, 26 nL, 27 nL, 28 nL, 29 nL, or 30 nL of each solution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the *E*. *coli* cells*, Bacillus* cells, *Trichoderma* cells, *Komagataella* cells, *Aspergillus* cells, *Thermothelomyces* cells, or *Saccharomyces* cells present in the picked colonies to multiply;
(h) isolating DNA from the *E. coli* cells, *Bacillus* cells, *Trichoderma* cells, *Komagataella* cells, *Aspergillus* cells, *Thermothelomyces* cells, or *Saccharomyces* cells in the liquid medium; and,
(i) sequencing the DNA encoding the enzyme.

Further, the invention provides a method for analyzing a library comprising, in sequence, the steps of:
(a) providing a library comprising a plurality of members, wherein each member comprises one or more *E. coli* cells, and wherein each member recombinantly expresses an enzyme selected from the group consisting of phytase, protease, beta-glucanase, xylanase, mannanase, lipase, cellulase, glucoamylase, amylase, alpha-amylase, and beta-amylase;
(b) arraying solutions of the members of the library in one or more microtiter plates;
(c) transferring a specified volume being 1 nL, 2 nL, 3 nL, 4 nL, 5 nL, 6 nL, 7 nL, 8 nL,9 nL, 10 nL, 11 nL, 12 nL, 13 nL, 14 nL, 15 nL,16 nL, 17 nL, 18 nL, 19 nL, 20 nL, 21 nL, 22 nL, 23 nL, 24 nL, 25 nL, 26 nL, 27 nL, 28 nL, 29 nL, or 30 nL of each solution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the *E*. *coli* cells present in the picked colonies to multiply;
(h) isolating DNA from the *E*. *coli* cells in the liquid medium; and
(i) sequencing the DNA encoding the enzyme.

Further, the invention provides a method for analyzing a library comprising, in sequence, the steps of:
(a) providing a library comprising a plurality of members, wherein each member comprises one or more *Komagataella phaffii* cells*,* and wherein each member recombinantly expresses an enzyme selected from the group consisting of phytase, protease, beta-glucanase, xylanase, mannanase, lipase, cellulase, glucoamylase, amylase, alpha-amylase, and beta-amylase;
(b) arraying solutions of the members of the library in one or more microtiter plates;
(c) transferring a specified volume being 1 nL, 2 nL, 3 nL, 4 nL, 5 nL, 6 nL, 7 nL, 8 nL,9 nL, 10 nL, 11 nL, 12 nL, 13 nL, 14 nL, 15 nL,16 nL, 17 nL, 18 nL, 19 nL, 20 nL, 21 nL, 22 nL, 23 nL, 24 nL, 25 nL, 26 nL, 27 nL, 28 nL, 29 nL, or 30 nL of each solution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the *Komagataella phaffii* cells present in the picked colonies to multiply;
(h) isolating DNA from the *Komagataella phaffii* cells in the liquid medium; and
(i) sequencing the DNA encoding the enzyme.

In another embodiment, in any of the preceding methods and embodiments in which a specified volume being 1 nL, 2 nL, 3 nL, 4 nL, 5 nL, 6 nL, 7 nL, 8 nL,9 nL, 10 nL, 11 nL, 12 nL, 13 nL, 14 nL, 15 nL,16 nL, 17 nL, 18 nL, 19 nL, 20 nL, 21 nL, 22 nL, 23 nL, 24 nL, 25 nL, 26 nL, 27 nL, 28 nL, 29 nL, or 30 nL is transferred, the protein has a desired function selected from the group consisting of an enzymatic activity, a substrate specificity, an enzymatic stability, an expression titer, and any combination thereof.

The number of wells in the one or more microtiter plates of any of the above methods or embodiments in which a specified volume being 1 nL, 2 nL, 3 nL, 4 nL, 5 nL, 6 nL, 7 nL, 8 nL,9 nL, 10 nL, 11 nL, 12 nL, 13 nL, 14 nL, 15 nL,16 nL, 17 nL, 18 nL, 19 nL, 20 nL, 21 nL, 22 nL, 23 nL, 24 nL, 25 nL, 26 nL, 27 nL, 28 nL, 29 nL, or 30 nL is transferred in step (c) and in which the one or more microtiter plates comprise wells may be, e.g., 6, 12, 24, 48, 96, 384, or 1536.

In an embodiment, in step (b) of any one of the inventive methods in which a specified volume being 1 nL, 2 nL, 3 nL, 4 nL, 5 nL, 6 nL, 7 nL, 8 nL,9 nL, 10 nL, 11 nL, 12 nL, 13 nL, 14 nL, 15 nL,16 nL, 17 nL, 18 nL, 19 nL, 20 nL, 21 nL, 22 nL, 23 nL, 24 nL, 25 nL, 26 nL, 27 nL, 28 nL, 29 nL, or 30 nL is transferred in step (c), dilutions of the solutions are prepared before the members are arrayed, optionally wherein the dilutions are serial dilutions. Dilutions can be performed in liquid growth media, buffers, or ultrapure water (e.g. Milli-Q water).

Accordingly, the invention provides a method for analyzing a library comprising, in sequence, the steps of:
(a) providing a library comprising a plurality of members, wherein each member comprises one or more cells, and wherein each member recombinantly expresses a protein;
(b) preparing dilutions of solutions of the members of the library and arraying the solutions and dilutions in one or more microtiter plates, optionally comprising wells;
(c) transferring a specified volume being 1 nL, 2 nL, 3 nL, 4 nL, 5 nL, 6 nL, 7 nL, 8 nL,9 nL, 10 nL, 11 nL, 12 nL, 13 nL, 14 nL, 15 nL,16 nL, 17 nL, 18 nL, 19 nL, 20 nL, 21 nL, 22 nL, 23 nL, 24 nL, 25 nL, 26 nL, 27 nL, 28 nL, 29 nL, or 30 nL of each solution and dilution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the cells present in the picked colonies to multiply;
(h) isolating DNA from the cells in the liquid medium; and,
(i) sequencing the DNA encoding the protein of interest.

Further, the invention provides a method for analyzing a library comprising, in sequence, the steps of:
(a) providing a library comprising a plurality of members, wherein each member comprises one or more cells, and wherein each member recombinantly expresses a protein;
(b) preparing serial dilutions of solutions of the members of the library and arraying the solutions and serial dilutions in one or more microtiter plates, optionally comprising wells;
(c) transferring a specified volume being 1 nL, 2 nL, 3 nL, 4 nL, 5 nL, 6 nL, 7 nL, 8 nL,9 nL, 10 nL, 11 nL, 12 nL, 13 nL, 14 nL, 15 nL,16 nL, 17 nL, 18 nL, 19 nL, 20 nL, 21 nL, 22 nL, 23 nL, 24 nL, 25 nL, 26 nL, 27 nL, 28 nL, 29 nL, or 30 nL of each solution and serial dilution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the cells present in the picked colonies to multiply;
(h) isolating DNA from the cells in the liquid medium; and,
(i) sequencing the DNA encoding the protein of interest.

Further, the invention provides a method for analyzing a library comprising, in sequence, the steps of:
(a) providing a library comprising a plurality of members, wherein each member comprises one or more cells, and wherein each member recombinantly expresses an enzyme, a peptide, an antibody or antigen-binding fragment thereof, a protein antibiotic, a fusion protein, a vaccine or a vaccine-like protein or particle, a growth factor, a hormone, or a cytokine;
(b) preparing dilutions of solutions of the members of the library and arraying the solutions and dilutions in one or more microtiter plates, optionally comprising wells;
(c) transferring a specified volume being 1 nL, 2 nL, 3 nL, 4 nL, 5 nL, 6 nL, 7 nL, 8 nL,9 nL, 10 nL, 11 nL, 12 nL, 13 nL, 14 nL, 15 nL,16 nL, 17 nL, 18 nL, 19 nL, 20 nL, 21 nL, 22 nL, 23 nL, 24 nL, 25 nL, 26 nL, 27 nL, 28 nL, 29 nL, or 30 nL each solution and dilution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the cells present in the picked colonies to multiply;
(h) isolating DNA from the cells in the liquid medium; and,
(i) sequencing the DNA encoding the protein of interest.

Further, the invention provides a method for analyzing a library comprising, in sequence, the steps of:
(a) providing a library comprising a plurality of members, wherein each member comprises one or more cells, and wherein each member recombinantly expresses an enzyme, a peptide, an antibody or antigen-binding fragment thereof, a protein antibiotic, a fusion protein, a vaccine or a vaccine-like protein or particle, a growth factor, a hormone, or a cytokine;
(b) preparing serial dilutions of solutions of the members of the library and arraying the solutions and serial dilutions in one or more microtiter plates, optionally comprising wells;
(c) transferring a specified volume being 1 nL, 2 nL, 3 nL, 4 nL, 5 nL, 6 nL, 7 nL, 8 nL,9 nL, 10 nL, 11 nL, 12 nL, 13 nL, 14 nL, 15 nL,16 nL, 17 nL, 18 nL, 19 nL, 20 nL, 21 nL, 22 nL, 23 nL, 24 nL, 25 nL, 26 nL, 27 nL, 28 nL, 29 nL, or 30 nL of each solution and serial dilution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the cells present in the picked colonies to multiply;
(h) isolating DNA from the cells in the liquid medium; and,
(i) sequencing the DNA encoding the protein of interest.

Further, the invention provides a method for analyzing a library comprising, in sequence, the steps of:
(a) providing a library comprising a plurality of members, wherein each member comprises one or more cells, and wherein each member recombinantly expresses an enzyme selected from the group consisting of phytase, protease, beta-glucanase, xylanase, mannanase, lipase, cellulase, glucoamylase, amylase, alpha-amylase, and beta-amylase;
(b) preparing dilutions of solutions of the members of the library and arraying the solutions and dilutions in one or more microtiter plates, optionally comprising wells;
(c) transferring a specified volume being 1 nL, 2 nL, 3 nL, 4 nL, 5 nL, 6 nL, 7 nL, 8 nL,9 nL, 10 nL, 11 nL, 12 nL, 13 nL, 14 nL, 15 nL,16 nL, 17 nL, 18 nL, 19 nL, 20 nL, 21 nL, 22 nL, 23 nL, 24 nL, 25 nL, 26 nL, 27 nL, 28 nL, 29 nL, or 30 nL of each solution and dilution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the cells present in the picked colonies to multiply;
(h) isolating DNA from the cells in the liquid medium; and,
(i) sequencing the DNA encoding the enzyme.

Further, the invention provides a method for analyzing a library comprising, in sequence, the steps of:
(a) providing a library comprising a plurality of members, wherein each member comprises one or more cells, and wherein each member recombinantly expresses an enzyme selected from the group consisting of phytase, protease, beta-glucanase, xylanase, mannanase, lipase, cellulase, glucoamylase, amylase, alpha-amylase, and beta-amylase;
(b) preparing serial dilutions of solutions of the members of the library and arraying the solutions and serial dilutions in one or more microtiter plates, optionally comprising wells;
(c) transferring a specified volume being 1 nL, 2 nL, 3 nL, 4 nL, 5 nL, 6 nL, 7 nL, 8 nL,9 nL, 10 nL, 11 nL, 12 nL, 13 nL, 14 nL, 15 nL,16 nL, 17 nL, 18 nL, 19 nL, 20 nL, 21 nL, 22 nL, 23 nL, 24 nL, 25 nL, 26 nL, 27 nL, 28 nL, 29 nL, or 30 nL of each solution and serial dilution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the cells present in the picked colonies to multiply;
(h) isolating DNA from the cells in the liquid medium; and,
(i) sequencing the DNA encoding the enzyme.

In an embodiment, in any of the preceding methods in which a specified volume being 1 nL, 2 nL, 3 nL, 4 nL, 5 nL, 6 nL, 7 nL, 8 nL,9 nL, 10 nL, 11 nL, 12 nL, 13 nL, 14 nL, 15 nL,16 nL, 17 nL, 18 nL, 19 nL, 20 nL, 21 nL, 22 nL, 23 nL, 24 nL, 25 nL, 26 nL, 27 nL, 28 nL, 29 nL, or 30 nL is transferred in step (c), and in which dilutions or serial dilutions are performed in step (b), the one or more cells may be selected from the group consisting of prokaryotic cells and eukaryotic cells. That is, each member of the library to be analyzed comprises one or more prokaryotic cells or eukaryotic cells. In one such embodiment, the one or more cells are prokaryotic cells and the prokaryotic cells are bacterial cells, i.e. each member of the library to be analyzed comprises one or more bacterial cells. In another such embodiment, the one or more cells are eukaryotic cells, and the eukaryotic cells are selected from the group consisting of fungal cells, yeast cells, animal cells, and plant cells, i.e. each member of the library to be analyzed comprises one or more fungal cells, yeast cells, animal cells, or plant cells.

Further, the invention provides a method for analyzing a library comprising, in sequence, the steps of:
(a) providing a library comprising a plurality of members, wherein each member comprises one or more *E*. *coli* cells*, Bacillus* cells, *Trichoderma* cells, *Komagataella* cells, *Aspergillus* cells, *Thermothelomyces* cells, or *Saccharomyces* cells, and wherein each member recombinantly expresses a protein;
(b) preparing dilutions of solutions of the members of the library and arraying the solutions and dilutions in one or more microtiter plates, optionally comprising wells;
(c) transferring a specified volume being 1 nL, 2 nL, 3 nL, 4 nL, 5 nL, 6 nL, 7 nL, 8 nL,9 nL, 10 nL, 11 nL, 12 nL, 13 nL, 14 nL, 15 nL,16 nL, 17 nL, 18 nL, 19 nL, 20 nL, 21 nL, 22 nL, 23 nL, 24 nL, 25 nL, 26 nL, 27 nL, 28 nL, 29 nL, or 30 nL of each solution and dilution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the *E*. *coli* cells*, Bacillus* cells, *Trichoderma* cells, *Komagataella* cells, *Aspergillus* cells, *Thermothelomyces* cells, or *Saccharomyces* cells present in the picked colonies to multiply;
(h) isolating DNA from the *E*. *coli* cells, *Bacillus* cells, *Trichoderma* cells, *Komagataella* cells, *Aspergillus* cells, *Thermothelomyces* cells, or *Saccharomyces* cells in the liquid medium; and,
(i) sequencing the DNA encoding the protein of interest.

Further, the invention provides a method for analyzing a library comprising, in sequence, the steps of:
(a) providing a library comprising a plurality of members, wherein each member comprises one or more *E*. *coli* cells, *Bacillus* cells, *Trichoderma* cells, *Komagataella* cells, *Aspergillus* cells, *Thermothelomyces* cells, or *Saccharomyces* cells, and wherein each member recombinantly expresses a protein;
(b) preparing serial dilutions of solutions of the members of the library and arraying the solutions and serial dilutions in one or more microtiter plates, optionally comprising wells;
(c) transferring a specified volume being 1 nL, 2 nL, 3 nL, 4 nL, 5 nL, 6 nL, 7 nL, 8 nL,9 nL, 10 nL, 11 nL, 12 nL, 13 nL, 14 nL, 15 nL,16 nL, 17 nL, 18 nL, 19 nL, 20 nL, 21 nL, 22 nL, 23 nL, 24 nL, 25 nL, 26 nL, 27 nL, 28 nL, 29 nL, or 30 nL of each solution and serial dilution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the *E*. *coli* cells, *Bacillus* cells, *Trichoderma* cells, *Komagataella* cells, *Aspergillus* cells, *Thermothelomyces* cells, or *Saccharomyces* cells present in the picked colonies to multiply;
(h) isolating DNA from the *E*. *coli* cells, *Bacillus* cells, *Trichoderma* cells, *Komagataella* cells, *Aspergillus* cells, *Thermothelomyces* cells, or *Saccharomyces* cells, Thermothelomyces cells, or Saccharomyces cells in the liquid medium; and,
(i) sequencing the DNA encoding the protein of interest.

Further, the invention provides a method for analyzing a library comprising, in sequence, the steps of:
(a) providing a library comprising a plurality of members, wherein each member comprises one or more *E*. *coli* cells, *Bacillus* cells, *Trichoderma* cells, *Komagataella* cells, *Aspergillus* cells, *Thermothelomyces* cells, or *Saccharomyces* cells, and wherein each member recombinantly expresses an enzyme, a peptide, an antibody or antigen-binding fragment thereof, a protein antibiotic, a fusion protein, a vaccine or a vaccine-like protein or particle, a growth factor, a hormone, or a cytokine;
(b) preparing dilutions of solutions of the members of the library and arraying the solutions and dilutions in one or more microtiter plates, optionally comprising wells;
(c) transferring a specified volume being 1 nL, 2 nL, 3 nL, 4 nL, 5 nL, 6 nL, 7 nL, 8 nL,9 nL, 10 nL, 11 nL, 12 nL, 13 nL, 14 nL, 15 nL,16 nL, 17 nL, 18 nL, 19 nL, 20 nL, 21 nL, 22 nL, 23 nL, 24 nL, 25 nL, 26 nL, 27 nL, 28 nL, 29 nL, or 30 nL of each solution and dilution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the *E*. *coli* cells*, Bacillus* cells, *Trichoderma* cells, *Komagataella* cells, *Aspergillus* cells, *Thermothelomyces* cells, or *Saccharomyces* cells present in the picked colonies to multiply;
(h) isolating DNA from the *E*. *coli* cells, *Bacillus* cells, *Trichoderma* cells, *Komagataella* cells, *Aspergillus* cells, *Thermothelomyces* cells, or *Saccharomyces* cells in the liquid medium; and,
(i) sequencing the DNA encoding the protein of interest.

Further, the invention provides a method for analyzing a library comprising, in sequence, the steps of:
(a) providing a library comprising a plurality of members, wherein each member comprises one or more *E*. *coli* cells, *Bacillus* cells, *Trichoderma* cells, *Komagataella* cells, *Aspergillus* cells, *Thermothelomyces* cells, or *Saccharomyces* cells, and wherein each member recombinantly expresses an enzyme, a peptide, an antibody or antigen-binding fragment thereof, a protein antibiotic, a fusion protein, a vaccine or a vaccine-like protein or particle, a growth factor, a hormone, or a cytokine;
(b) preparing serial dilutions of solutions of the members of the library and arraying the solutions and serial dilutions in one or more microtiter plates, optionally comprising wells;
(c) transferring a specified volume being 1 nL, 2 nL, 3 nL, 4 nL, 5 nL, 6 nL, 7 nL, 8 nL,9 nL, 10 nL, 11 nL, 12 nL, 13 nL, 14 nL, 15 nL,16 nL, 17 nL, 18 nL, 19 nL, 20 nL, 21 nL, 22 nL, 23 nL, 24 nL, 25 nL, 26 nL, 27 nL, 28 nL, 29 nL, or 30 nL of each solution and serial dilution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the *E*. *coli* cells, *Bacillus* cells, *Trichoderma* cells, *Komagataella* cells, *Aspergillus* cells, *Thermothelomyces* cells, or *Saccharomyces* cells present in the picked colonies to multiply;
(h) isolating DNA from the *E*. *coli* cells, *Bacillus* cells, *Trichoderma* cells, *Komagataella* cells, *Aspergillus* cells, *Thermothelomyces* cells, or *Saccharomyces* cells in the liquid medium; and,
(i) sequencing the DNA encoding the protein of interest.

Further, the invention provides a method for analyzing a library comprising, in sequence, the steps of:
(a) providing a library comprising a plurality of members, wherein each member comprises one or more *E*. *coli* cells*, Bacillus* cells, *Trichoderma* cells, *Komagataella* cells, *Aspergillus* cells, *Thermothelomyces* cells, or *Saccharomyces* cells, and wherein each member recombinantly expresses an enzyme selected from the group consisting of phytase, protease, beta-glucanase, xylanase, mannanase, lipase, cellulase, glucoamylase, amylase, alpha-amylase, and beta-amylase;
(b) preparing dilutions of solutions of the members of the library and arraying the solutions and dilutions in one or more microtiter plates, optionally comprising wells;
(c) transferring a specified volume being 1 nL, 2 nL, 3 nL, 4 nL, 5 nL, 6 nL, 7 nL, 8 nL,9 nL, 10 nL, 11 nL, 12 nL, 13 nL, 14 nL, 15 nL, 16 nL, 17 nL, 18 nL, 19 nL, 20 nL, 21 nL, 22 nL, 23 nL, 24 nL, 25 nL, 26 nL, 27 nL, 28 nL, 29 nL, or 30 nL of each solution and dilution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the *E*. *coli* cells, *Bacillus* cells, *Trichoderma* cells, *Komagataella* cells, *Aspergillus* cells, *Thermothelomyces* cells, or *Saccharomyces* cells present in the picked colonies to multiply;
(h) isolating DNA from the *E*. *coli* cells*, Bacillus* cells, *Trichoderma* cells, *Komagataella* cells, *Aspergillus* cells, *Thermothelomyces* cells, or *Saccharomyces* cells in the liquid medium; and,
(i) sequencing the DNA encoding the enzyme.

Further, the invention provides a method for analyzing a library comprising, in sequence, the steps of:
(a) providing a library comprising a plurality of members, wherein each member comprises one or more *E*. *coli* cells, and wherein each member recombinantly expresses an enzyme selected from the group consisting of phytase, protease, beta-glucanase, xylanase, mannanase, lipase, cellulase, glucoamylase, amylase, alpha-amylase, and beta-amylase;
(b) preparing dilutions of solutions of the members of the library and arraying the solutions and dilutions in one or more microtiter plates, optionally comprising wells;
(c) transferring a specified volume being 1 nL, 2 nL, 3 nL, 4 nL, 5 nL, 6 nL, 7 nL, 8 nL,9 nL, 10 nL, 11 nL, 12 nL, 13 nL, 14 nL, 15 nL,16 nL, 17 nL, 18 nL, 19 nL, 20 nL, 21 nL, 22 nL, 23 nL, 24 nL, 25 nL, 26 nL, 27 nL, 28 nL, 29 nL, or 30 nL of each solution and dilution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the *E*. *coli* cells present in the picked colonies to multiply;
(h) isolating DNA from the *E*. *coli* cells in the liquid medium; and
(i) sequencing the DNA encoding the enzyme.

Further, the invention provides a method for analyzing a library comprising, in sequence, the steps of:
(a) providing a library comprising a plurality of members, wherein each member comprises one or more *Komagataella phaffii* cells, and wherein each member recombinantly expresses an enzyme selected from the group consisting of phytase, protease, beta-glucanase, xylanase, mannanase, lipase, cellulase, glucoamylase, amylase, alpha-amylase, and beta-amylase;
(b) preparing dilutions of solutions of the members of the library and arraying the solutions and dilutions in one or more microtiter plates, optionally comprising wells;
(c) transferring a specified volume being 1 nL, 2 nL, 3 nL, 4 nL, 5 nL, 6 nL, 7 nL, 8 nL,9 nL, 10 nL, 11 nL, 12 nL, 13 nL, 14 nL, 15 nL,16 nL, 17 nL, 18 nL, 19 nL, 20 nL, 21 nL, 22 nL, 23 nL, 24 nL, 25 nL, 26 nL, 27 nL, 28 nL, 29 nL, or 30 nL of each solution and dilution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the *Komagataella phaffii* cells present in the picked colonies to multiply;
(h) isolating DNA from the *Komagataella phaffii* cells in the liquid medium; and,
(i) sequencing the DNA encoding the enzyme.

Further, the invention provides a method for analyzing a library comprising, in sequence, the steps of:
(a) providing a library comprising a plurality of members, wherein each member comprises one or more *E*. *coli* cells, *Bacillus* cells, *Trichoderma* cells, *Komagataella* cells, *Aspergillus* cells, *Thermothelomyces* cells, or *Saccharomyces* cells, and wherein each member recombinantly expresses an enzyme selected from the group consisting of phytase, protease, beta-glucanase, xylanase, mannanase, lipase, cellulase, glucoamylase, amylase, alpha-amylase, and beta-amylase;
(b) preparing serial dilutions of solutions of the members of the library and arraying the solutions and serial dilutions in one or more microtiter plates, optionally comprising wells;
(c) transferring a specified volume being 1 nL, 2 nL, 3 nL, 4 nL, 5 nL, 6 nL, 7 nL, 8 nL,9 nL, 10 nL, 11 nL, 12 nL, 13 nL, 14 nL, 15 nL,16 nL, 17 nL, 18 nL, 19 nL, 20 nL, 21 nL, 22 nL, 23 nL, 24 nL, 25 nL, 26 nL, 27 nL, 28 nL, 29 nL, or 30 nL of each solution and serial dilution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the *E*. *coli* cells*, Bacillus* cells, *Trichoderma* cells, *Komagataella* cells, *Aspergillus* cells, *Thermothelomyces* cells, or *Saccharomyces* cells present in the picked colonies to multiply;
(h) isolating DNA from the *E*. *coli* cells, *Bacillus* cells, *Trichoderma* cells, *Komagataella* cells, *Aspergillus* cells, *Thermothelomyces* cells, or *Saccharomyces* cells in the liquid medium; and,
(i) sequencing the DNA encoding the enzyme.

Further, the invention provides a method for analyzing a library comprising, in sequence, the steps of:
(a) providing a library comprising a plurality of members, wherein each member comprises one or more *E*. *coli* cells, and wherein each member recombinantly expresses an enzyme selected from the group consisting of phytase, protease, beta-glucanase, xylanase, mannanase, lipase, cellulase, glucoamylase, amylase, alpha-amylase, and beta-amylase;
(b) preparing serial dilutions of solutions of the members of the library and arraying the solutions and serial dilutions in one or more microtiter plates, optionally comprising wells;
(c) transferring a specified volume being 1 nL, 2 nL, 3 nL, 4 nL, 5 nL, 6 nL, 7 nL, 8 nL,9 nL, 10 nL, 11 nL, 12 nL, 13 nL, 14 nL, 15 nL,16 nL, 17 nL, 18 nL, 19 nL, 20 nL, 21 nL, 22 nL, 23 nL, 24 nL, 25 nL, 26 nL, 27 nL, 28 nL, 29 nL, or 30 nL of each solution and serial dilution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the *E*. *coli* cells present in the picked colonies to multiply;
(h) isolating DNA from the *E*. *coli* cells in the liquid medium; and,
(i) sequencing the DNA encoding the enzyme.

Further, the invention provides a method for analyzing a library comprising, in sequence, the steps of:
(a) providing a library comprising a plurality of members, wherein each member comprises one or more *Komagataella phaffii* cells, and wherein each member recombinantly expresses an enzyme selected from the group consisting of phytase, protease, beta-glucanase, xylanase, mannanase, lipase, cellulase, glucoamylase, amylase, alpha-amylase, and beta-amylase;
(b) preparing serial dilutions of solutions of the members of the library and arraying the solutions and serial dilutions in one or more microtiter plates, optionally comprising wells;
(c) transferring a specified volume being 1 nL, 2 nL, 3 nL, 4 nL, 5 nL, 6 nL, 7 nL, 8 nL,9 nL, 10 nL, 11 nL, 12 nL, 13 nL, 14 nL, 15 nL,16 nL, 17 nL, 18 nL, 19 nL, 20 nL, 21 nL, 22 nL, 23 nL, 24 nL, 25 nL, 26 nL, 27 nL, 28 nL, 29 nL, or 30 nL of each solution and serial dilution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the *Komagataella phaffii* cells present in the picked colonies to multiply;
(h) isolating DNA from the *Komagataella phaffii* cells in the liquid medium; and,
(i) sequencing the DNA encoding the enzyme.

The number of wells in the one or more microtiter plates of any of the above methods or embodiments in which the one or more microtiter plates comprise wells may be, e.g., 6, 12, 24, 48, 96, 384, or 1536.

In an embodiment, in any of the preceding methods and embodiments in which a specified volume being 1 nL, 2 nL, 3 nL, 4 nL, 5 nL, 6 nL, 7 nL, 8 nL,9 nL, 10 nL, 11 nL, 12 nL, 13 nL, 14 nL, 15 nL,16 nL, 17 nL, 18 nL, 19 nL, 20 nL, 21 nL, 22 nL, 23 nL, 24 nL, 25 nL, 26 nL, 27 nL, 28 nL, 29 nL, or 30 nL is transferred in step (c) and in which dilutions or serial dilutions are performed in step (b), the protein has a desired function selected from the group consisting of an enzymatic activity, a substrate specificity, an enzymatic stability, an expression titer, and any combination thereof.

In an embodiment, in any of the above methods or embodiments in which a specified volume being 1 nL, 2 nL, 3 nL, 4 nL, 5 nL, 6 nL, 7 nL, 8 nL,9 nL, 10 nL, 11 nL, 12 nL, 13 nL, 14 nL, 15 nL,16 nL, 17 nL, 18 nL, 19 nL, 20 nL, 21 nL, 22 nL, 23 nL, 24 nL, 25 nL, 26 nL, 27 nL, 28 nL, 29 nL, or 30 nL is transferred in step (c), the solutions and/or dilutions of the solutions comprise a liquid comprising a glycerol stock. The glycerol stock may comprise, e.g. 10%-60%, preferably 17-30%, by volume of glycerol.

In an embodiment, in step (c) of any of the inventive methods and embodiments described above, the one or more agar trays is preferably located above the one or more microtiter plates, and the one or more agar trays are oriented such that their agar surface faces the one or more microtiter plates.

In an embodiment, the incubation time in step (d) of any of the inventive methods and embodiments described above is from 12 hours to 120 hours.

In an embodiment, the incubation temperature in step (d) of any of the inventive methods and embodiments described above is from 15 degrees C to 50 degrees C.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

The detailed description is merely exemplary in nature and is not intended to limit application and uses. The following examples further illustrate the present invention without, however, limiting the scope of the invention thereto. Various changes and modifications can be made by those skilled in the art on the basis of the description of the invention, and such changes and modifications are also included in the present invention.

### Examples

### 1. Manual isolation of single P. pastoris colonies

Three *Pichia pastoris* strains containing unique amylase variants, BD51541, BD51542, and BD51546, were streaked from frozen glycerol-stocks onto YPD-Zeo¹⁰⁰ agar plates and grown for three days at 30°C. Single colonies from each plate were used to inoculate 4 mL YPD-Zeo¹⁰⁰ medium in 15 mL culture tubes. Cultures were grown at 30°C with shaking at 250 rpm for 18-24 hours.

### 2. General protocol for automated isolation of single colonies

1. An ATS source plate is created on an 8-channel Hamilton liquid handler:
   - Library members are arrayed from source glycerol stock plates using a worklist
   - A serial dilution of each library member is created in an ATS compatible 384 flat bottom plate. If an intermediate plate is needed for high dilution factors, a 96 deep well plate is used.
2. A map file is created for the ATS run - defining mapping of source to destination spots, as well as volume of the spots to be transferred onto the destination agar tray. In the software, the destination plate is defined as a 3456 plate (72x48 wells). This size grid allows for a pattern similar to a 96 well plate, where each well is one variant spotted in its own sub-grid of 25 spots (5x5). A spot is 20 nL. Plating in this format later facilitates automated picking on a colony picker, using each of the 96 regions as a picking zone.
3. To ensure single colonies, 3 different dilutions of the same library member are spotted side by side. With this layout, the capacity of an OmniTray™ is 32 library members.
4. The source plate and a destination agar containing OmniTray™ are loaded on to the ATS. Using an appropriate map file, 6 columns of the source plate are spotted on to one destination tray.
5. The agar tray is then lidded, placed in an incubator, and left to grow. The growth conditions, e.g. temperature and duration, depend on the nature of the library members. Common settings are 30 °C overnight for *E*. *coli* or is 30 °C for 48 hours for *P. pastoris.*

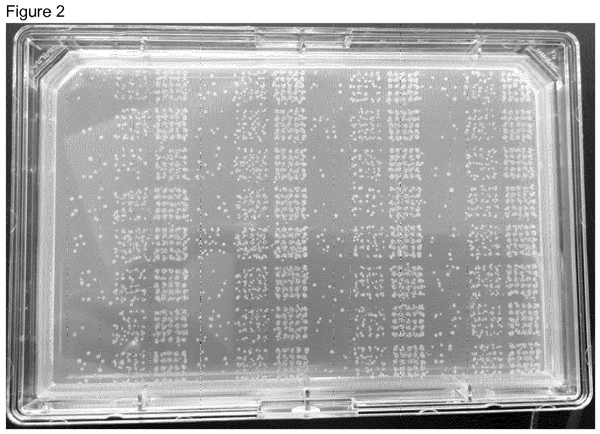

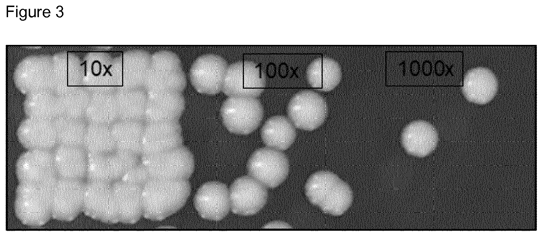

An exemplary result (*E. coli*) can be seen in Figure 2. A close-up of a further exemplary result (*P. pastoris*) can be seen in Figure 3.

### 3. Automated isolation and sequencing of single P. pastoris colonies

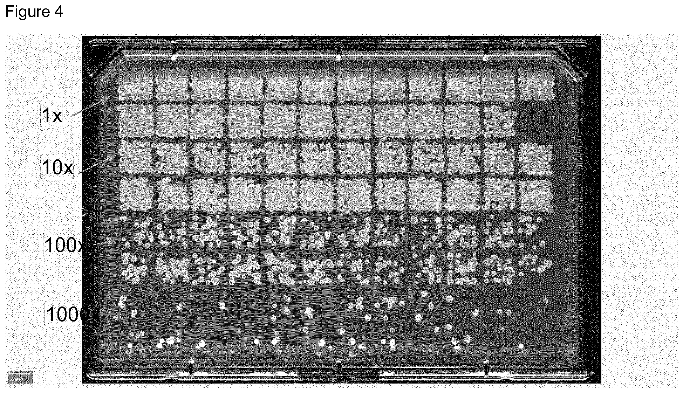

Single colonies were isolated according to the general protocol of Example 2, unless otherwise specified in the following: An ATS source plate was created on an 8-channel Hamilton liquid handler using the same three frozen glycerol stocks as in Example 1 as well as an equal mixture of the three cultures obtained in Example 1 (generated by normalizing each of the three cultures based on OD₆₀₀ and then combining equal volumes of each) by arraying them into an ATS (EDS Biosystems) compatible 384 flat bottom plate (384 Greiner 781090) using a worklist. Serial dilutions up to 100,000x (six total dilutions) were performed by the liquid handler in the same microplate. A map file was created according to Example 2, but to ensure single colonies, all 6 different dilutions (undiluted, 10x, 100x, 1000x, 10000x, 100000x) of each of the four *P*. *pastoris were* transferred onto YPD-Zeo¹⁰⁰ containing Omni-Tray™ destination agar trays using the ATS. The agar trays were lidded, placed in an incubator, and grown at 30 °C for 48 hours (see Figure 4; second plate not pictured due to no colonies growing at the higher dilutions).

### 4. Sequencing of genes of interest from single colonies.

Culture PCR amplifying full-length amylase genes was performed using each independent culture obtained in Example 1, or the equal mixture of each of the three cultures used in Example 2, as the template. Colony PCR amplifying full-length amylase gene was performed for multiple colonies from each dilution set obtained in Example 3. The amylase gene product from these PCR reactions were sequenced by GENEWIZ (New Jersey) and analyzed using Geneious® software.

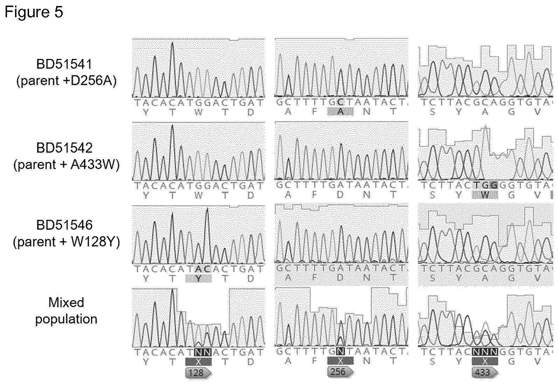

All three sequences from cultures derived from single colonies obtained in Example 1 show clean, high quality sequencing data for the gene (see Figure 5). As expected, sequence from the mixed-population culture shows high quality sequence throughout the gene with the exception of three amino acid residues; 128, 256, and 433 (see Figure 5). These residues are sites where the genes in the three *Pichia* strains differ from one another.

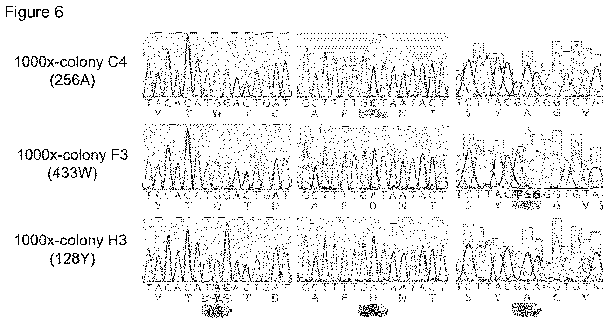

PCRs for single colonies from the 1x, 10x, and 100x dilution arrays obtained in Example 3 showed at least some mixed sequences. All colonies from 1,000x or higher dilution arrays resulted in non-mixed sequences, indicating that each colony was a homogeneous clonal population. Each of the three different strains were represented among the 40 isolated colonies sequenced from the 1,000x dilution (see Figure 6).

## Claims

1. A method for analyzing a library comprising, in sequence, the steps of:
(a) providing a library comprising a plurality of members, wherein each member comprises one or more cells, and wherein each member recombinantly expresses a protein of interest;
(b) arraying solutions of the members of the library in one or more microtiter plates;
(c) transferring a specified volume of each solution from the one or more microtiter plates onto one or more agar trays using an acoustic dispenser;
(d) incubating the one or more agar trays until colonies form;
(e) picking one or more single colonies for each member using a robot;
(f) inoculating each picked colony into liquid medium;
(g) incubating the inoculated liquid medium containing the picked colony, allowing the cells present in the picked colonies to multiply;
(h) isolating DNA from the cells in the liquid medium; and,
(i) sequencing the DNA encoding the protein of interest.

2. The method of claim 1, wherein the protein of interest is an enzyme, a peptide, an antibody or antigen-binding fragment thereof, a protein antibiotic, a fusion protein, a vaccine or a vaccine-like protein or particle, a growth factor, a hormone, or a cytokine.

3. The method of claim 2, wherein the protein of interest is an enzyme, preferably wherein the enzyme is selected from the group consisting of phytase, protease, beta-glucanase, xylanase, mannanase, lipase, cellulase, glucoamylase, amylase, alpha-amylase, and beta-amylase.

4. The method of any one of the preceding claims, wherein the one or more cells are selected from the group consisting of prokaryotic cells and eukaryotic cells.

5. The method of claim 4, wherein the one or more cells are prokaryotic cells and the prokaryotic cells are bacterial cells.

6. The method of claim 4, wherein the one or more cells are eukaryotic cells, and the eukaryotic cells are selected from the group consisting of fungal cells, yeast cells, animal cells, and plant cells.

7. The method of any one of claims 1 to 4, wherein the one or more cells are selected from the group consisting of *E*. *coli* cells*, Bacillus* cells, *Trichoderma* cells, *Komagataella* cells, *Aspergillus* cells, *Thermothelomyces* cells, and *Saccharomyces* cells.

8. The method of any one of the preceding claims, wherein the protein of interest has a desired function selected from the group consisting of an enzymatic activity, a substrate specificity, an enzymatic stability, an expression titer, and any combination thereof.

9. The method of any one of the preceding claims, wherein the one or more microtiter plates comprise wells, optionally wherein the number of wells is selected from the group consisting of 6, 12, 24, 48, 96, 384, and 1536.

10. The method of any one of the preceding claims, wherein in step (b) dilutions of the solutions are prepared before the members are arrayed, optionally wherein the dilutions are serial dilutions and wherein the solutions and the dilutions are arrayed in step (b).

11. The method of any one of the preceding claims, wherein the solutions and/or dilutions of the solutions comprise a liquid comprising a glycerol stock.

12. The method of any one of the preceding claims, wherein the specified volume transferred from the one or more microtiter plates onto the one or more agar trays using the acoustic dispenser in step (c) is 1 nL, 2 nL, 3 nL, 4 nL, 5 nL, 6 nL, 7 nL, 8 nL,9 nL, 10 nL, 11 nL, 12 nL, 13 nL, 14 nL, 15 nL,16 nL, 17 nL, 18 nL, 19 nL, 20 nL, 21 nL, 22 nL, 23 nL, 24 nL, 25 nL, 26 nL, 27 nL, 28 nL, 29 nL, or 30 nL.

13. The method of any one of the preceding claims, wherein in step (c), the one or more agar trays are located above the one or more microtiter plates, and wherein the one or more agar trays are oriented such that their agar surface faces the one or more microtiter plates.

14. The method of any one of the preceding claims, wherein the incubation time in step (d) is from 12 hours to 120 hours.

15. The method of any one of the preceding claims, wherein the incubation temperature in step (d) is from 15 degrees C to 50 degrees C.
